(19) Europäisches Patentamt
European Patent Office
Office européen des brevets

(11) **EP 3 944 003 B1**

(12) **EUROPEAN PATENT SPECIFICATION**

(45) Date of publication and mention of the grant of the patent:
**04.06.2025 Bulletin 2025/23**

(21) Application number: **20809183.5**

(22) Date of filing: **16.04.2020**

(51) International Patent Classification (IPC):
***G02C 7/04*** *(2006.01)* ***G02C 13/00*** *(2006.01)*
***A61L 2/04*** *(2006.01)* ***A61L 12/04*** *(2006.01)*
***A61L 29/04*** *(2006.01)* ***A61L 29/06*** *(2006.01)*
***A61L 29/14*** *(2006.01)* ***A61L 31/04*** *(2006.01)*
***A61L 31/06*** *(2006.01)* ***A61L 31/14*** *(2006.01)*
***A61L 27/16*** *(2006.01)* ***A61L 27/18*** *(2006.01)*
***A61L 27/34*** *(2006.01)* ***A61L 27/50*** *(2006.01)*
***A61L 27/52*** *(2006.01)* ***A61L 27/54*** *(2006.01)*

(52) Cooperative Patent Classification (CPC):
(C-Sets available)
**A61L 27/50; A61L 27/18; A61L 27/34; A61L 27/52; A61L 29/06; A61L 29/085; A61L 29/14; A61L 29/145;** A61L 2400/10; A61L 2420/02; B29D 11/00096; G02C 7/04 (Cont.)

(86) International application number:
**PCT/JP2020/016679**

(87) International publication number:
**WO 2020/235275 (26.11.2020 Gazette 2020/48)**

(54) **METHOD FOR MANUFACTURING MEDICAL DEVICE**

VERFAHREN ZUR HERSTELLUNG EINER MEDIZINISCHEN VORRICHTUNG

PROCÉDÉ DE FABRICATION DE DISPOSITIF MÉDICAL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.05.2019 JP 2019094294**

(43) Date of publication of application:
**26.01.2022 Bulletin 2022/04**

(73) Proprietor: **Toray Industries, Inc.**
**Tokyo 103-8666 (JP)**

(72) Inventors:
- **KITAGAWA, Rumiko**
  **Otsu-shi, Shiga 520-8558 (JP)**
- **NAKAMURA, Masataka**
  **Otsu-shi, Shiga 520-8558 (JP)**

(74) Representative: **Prüfer & Partner mbB**
**Patentanwälte · Rechtsanwälte**
**Sohnckestraße 12**
**81479 München (DE)**

(56) References cited:
**WO-A1-2017/169873**
**WO-A1-2018/073702**
**WO-A1-2019/031477**
**JP-A- 2011 197 196**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**A61L 27/18, C08L 83/04;**
**A61L 27/34, C08L 33/10;**
**A61L 29/06, C08L 83/04;**
**A61L 29/085, C08L 33/10**

**Description**

TECHNICAL FIELD

The present invention relates to a method for manufacturing a medical device.

BACKGROUND ART

Conventionally, devices containing resin soft materials such as silicone rubber and a hydrogel, and devices containing hard materials such as metals and glass are used for various applications in various fields.

Applications of the devices containing a soft material include medical devices intended for introduction into a living body or for covering a living body surface, biotechnological devices such as a cell culture sheet and a scaffold material for tissue regeneration, and cosmetic devices such as a facial pack.

Applications of the devices containing a hard material include use as an electrical appliance such as a personal computer, a mobile phone, and a display, use as an ampoule used in an injection drug, and use as a diagnosis/analysis tool such as a capillary tube and a biosensing chip.

In the case where any of various devices is introduced into a living body or attached to a living body surface as, for example, a medical device, it is important to modify the surface of a substrate of the medical device for the purpose of improving biocompatibility. If the surface modification can impart to the medical device better properties, for example, properties such as hydrophilicity, lubricity, biocompatibility, and drug efficacy than those before the surface modification, a user (patient or the like) can expect improvement in feeling of use, reduction in discomfort, improvement in symptoms, and the like.

Various methods are known for modifying the surface of a substrate of the medical device.

For example, Patent Document 1 discloses a method for imparting good water wettability to a surface of a substrate by heating the substrate in a solution containing a polymer having a hydroxyl group and having a pH of 2.0 or more and 6.0 or less.

In addition, there is known a method for improving the wearability of a substrate by subjecting the substrate to autoclave sterilization in a solution containing one or more types of polymer materials and having a pH in the range of 6 to 9 (see, for example, Patent Documents 2 to 4). In Patent Document 5, a polymer for surface treatment is described. The polymer is a water-soluble copolymer containing monomer A and monomer B as structural units. Monomer A has a (meth) acryloyl group and an ester structure or an amide structure in the molecule, wherein the ester structure and the amide structure do not contain any portions of the (meth)acryloyl group. Monomer B has a (meth)acrylic amide group in the molecule and has a structure different from that of monomer A. Further, a medical device having the polymer on a surface thereof is described. Further, a wetting agent containing the polymer is described.

PRIOR ART DOCUMENTS

PATENT DOCUMENTS

Patent Document 1: International Publication No. 2017/146102
Patent Document 2: Japanese Unexamined Patent Application Publication No. 2011-512546
Patent Document 3: Japanese Unexamined Patent Application Publication No. 2003-535626
Patent Document 4: Japanese Patent No. 5154231
Patent Document 5: WO 2017/169873 A1

SUMMARY OF THE INVENTION

PROBLEMS TO BE SOLVED BY THE INVENTION

Unfortunately, in the method for modifying the substrate surface as described in Patent Document 1, the solution containing the heated substrate has a pH of 6.0 or less. Therefore, in the case of using the substrate in, for example, a medical device such as an ophthalmic lens, it is necessary to additionally perform a washing step and a sterilization step in a neutral solution in order to eliminate irritation to the eye. These additional steps may lead to an increase in the manufacturing cost.

As for a contact lens as described in Patent Document 2, which is obtained by immersing a substrate in a packaging solution containing acid-terminated polyvinylpyrrolidone and subjecting the substrate to autoclave sterilization,

the document has no specific description on the durability of water wettability. Therefore, it is difficult to expect that the contact lens maintains sufficiently durable water wettability to withstand scrubbing and washing. In addition, although specific acid-terminated polyvinylpyrrolidone is prepared in the examples by a method of reacting hydroxyl-functionalized poly(vinylpyrrolidone) and poly(vinylpyrrolidone-co-allyl alcohol) as polymers with succinic anhydride, there is a problem that it is difficult to control the number of introduced acid terminals.

In the method for modifying the substrate surface as described in Patent Document **3,** as a result of specific study of such a surface treatment using poly(acrylic acid-co-acrylamide) and poly(acrylic acid-co-vinylpyrrolidone), the substrate surface is insufficient in performance such as hydrophilicity.

In the method for modifying the substrate surface as described in Patent Document 4, as a result of specific study of such a surface treatment using polyvinylpyrrolidone, the substrate surface is still insufficient in performance such as hydrophilicity.

The present invention has been made in view of the above-mentioned problems of the prior art. That is, an object of the present invention is to provide a method for easily manufacturing a medical device having highly durable hydrophilicity.

## SOLUTIONS TO THE PROBLEMS

In order to achieve the above-mentioned object, the present invention provides:

a method for manufacturing a medical device, the method including a step of heating an aqueous packaging solution, wherein the heating step is performed in a state in which the aqueous packaging solution contains at least one type of hydrophilic polymer, and a substrate of the medical device is at least partially in contact with the aqueous packaging solution, and
the method satisfies all of requirements (a) to (c) shown below:

(a) the hydrophilic polymer is a (co)polymer of a (meth) acrylamide having two substituents each having 2 or more carbon atoms on a nitrogen atom;
(b) the aqueous packaging solution has a mass% concentration of the hydrophilic polymer in a range of 0.0001 to 30 mass%; and
(c) the aqueous packaging solution has a pH in a range of 6.1 to 8.0 after the heating step.

## EFFECTS OF THE INVENTION

According to the present invention, unlike in the prior art, it is possible to easily provide a medical device having highly durable hydrophilicity.

## EMBODIMENTS OF THE INVENTION

A method for manufacturing a medical device of the present invention is a method including a step of heating an aqueous packaging solution,

wherein the heating step is performed in a state in which the aqueous packaging solution contains at least one type of hydrophilic polymer, and a substrate of the medical device is at least partially in contact with the aqueous packaging solution, and
the method satisfies all of requirements (a) to (c) shown below:

(a) the hydrophilic polymer is a (co)polymer of a (meth) acrylamide having two substituents each having 2 or more carbon atoms on a nitrogen atom;
(b) the aqueous packaging solution has a mass% concentration of the hydrophilic polymer in a range of 0.0001 to 30 mass%; and
(c) the aqueous packaging solution has a pH in a range of 6.1 to 8.0 after the heating step.

In the present invention, the "(meth)acrylamide having two substituents each having 2 or more carbon atoms on a nitrogen atom" refers to a (meth)acrylamide having, in addition to a (meth)acrylic group, two substituents each having 2 or more carbon atoms on a nitrogen atom.

In the present invention, the "(meth)acrylamide" includes both an acrylamide and a methacrylamide. This also applies to notations such as "(meth)acrylate" and "(meth)acrylic acid". The "(co)polymer" includes both a homopolymer and a copolymer.

In the present invention, the medical device may have a lens shape, and is preferably an ophthalmic lens. Examples of the medical device having a lens shape include ophthalmic lenses such as contact lenses, intraocular lenses, artificial corneas, corneal inlays, corneal onlays, and eyeglass lenses. An ophthalmic lens, particularly a contact lens, is one of the most preferred aspects of the present invention.

In the present invention, the medical device may have a tubular shape. Examples of the tubular medical device include infusion tubes, gas transport tubes, drainage tubes, blood circuits, covering tubes, catheters, stents, sheaths, tube connectors, access ports, and hollow fibers for heart-lung machines.

In the present invention, the medical device may have a sheet shape or a film shape. Examples of the sheet-shaped or film-shaped medical device include skin covering materials, wound covering materials, skin protective materials, drug carriers for skin, biosensor chips, and endoscope covering materials.

In the present invention, the medical device may have a storage container shape. Examples of the medical device having a storage container shape include drug carriers, cuffs, and drainage bags.

In the present invention, the medical device is preferably an ophthalmic lens, a skin covering material, a wound covering material, a skin protective material, a drug carrier for skin, an infusion tube, a gas transport tube, a drainage tube, a blood circuit, a covering tube, a catheter, a stent, a sheath biosensor chip, a heart-lung machine, or an endoscope covering material. The medical device is more preferably an ophthalmic lens.

In particular, it is one of the most preferred aspects of the present invention that the ophthalmic lens be a contact lens. In the present invention, the "contact lens" encompasses both contact lenses for vision correction purpose and contact lenses for cosmetic purpose.

In the present invention, as for the substrate of the medical device, either a water-containing substrate or a water-free substrate can be used. Examples of the material of the water-containing substrate include a hydrogel and a silicone hydrogel. Silicone hydrogels are particularly preferred because they have flexibility that gives excellent use comfort as well as high oxygen permeability. Examples of the material of the water-free substrate include a low water content soft material and a low water content hard material. That is, in the method for manufacturing a medical device of the present invention, the substrate preferably contains at least one type of material selected from the group consisting of a hydrogel, a silicone hydrogel, a low water content soft material, and a low water content hard material.

As for the material of the water-containing substrate, the present invention is applicable to both a general hydrogel containing no silicone and a hydrogel containing silicone (that is, a silicone hydrogel). The present invention can be particularly suitably used in a silicone hydrogel because the surface physical properties can be greatly improved.

Hereinafter, the United States Adopted Names (USAN) may be used to represent materials. In the USAN, symbols such as A, B, and C may be added at the end of materials to represent variations of the materials. In the present specification, any notation with no symbol added at the end of a material represents all the variations of the material. For example, a simple notation of "ocufilcon" represents all the variations of ocufilcon, such as "ocufilcon A", "ocufilcon B", "ocufilcon C", "ocufilcon D", "ocufilcon E", and "ocufilcon F".

In the method for manufacturing a medical device of the present invention, the hydrogel is preferably selected from the group consisting of tefilcon, tetrafilcon, helfilcon, mafilcon, polymacon, hioxifilcon, alfafilcon, omafilcon, nelfilcon, nesofilcon, hilafilcon, acofilcon, deltafilcon, etafilcon, focofilcon, ocufilcon, phemfilcon, methafilcon, and vilfilcon.

For example, when the hydrogel is contained in a contact lens, the hydrogel is classified into Group 1 to Group 4 of contact lens materials defined by the U.S. Food and Drug Administration (FDA). In particular, from the viewpoint of exhibiting good water wettability and antifouling properties, Group 2 and Group 4 are more preferred, and Group 4 is particularly preferred.

Group 1 represents nonionic hydrogel lens materials having a water content of less than 50 mass%. Specific examples thereof include tefilcon, tetrafilcon, helfilcon, mafilcon, polymacon, and hioxifilcon.

Group 2 represents nonionic hydrogel lens materials having a water content of 50 mass% or more. Specific examples thereof include alfafilcon, omafilcon, hioxifilcon, nelfilcon, nesofilcon, hilafilcon, and acofilcon. From the viewpoint of exhibiting good water wettability and antifouling properties, omafilcon, hioxifilcon, nelfilcon, and nesofilcon are more preferred, omafilcon and hioxifilcon are still more preferred, and omafilcon is particularly preferred.

Group 3 represents ionic hydrogel lens materials having a water content of less than 50 mass%. Specific examples thereof include deltafilcon.

Group 4 represents ionic hydrogel lens materials having a water content of 50 mass% or more. Specific examples thereof include etafilcon, focofilcon, ocufilcon, phemfilcon, methafilcon, and vilfilcon. From the viewpoint of exhibiting good water wettability and antifouling properties, etafilcon, focofilcon, ocufilcon, and phemfilcon are more preferred, etafilcon and ocufilcon are still more preferred, and etafilcon is particularly preferred.

For example, when the silicone hydrogel is contained in a contact lens, preferred specific examples of the silicone hydrogel include silicone hydrogels selected from the group belonging to Group 5 of contact lens materials defined by the U.S. Food and Drug Administration (FDA).

The silicone hydrogel is preferably a hydrophilic polymer containing a silicon atom in the main chain and/or the side chain, and examples thereof include a copolymer of a monomer containing a siloxane bond with a hydrophilic

monomer.

Specifically, the silicone hydrogel is preferably selected from the group consisting of lotrafilcon, galyfilcon, narafilcon, senofilcon, comfilcon, enfilcon, balafilcon, efrofilcon, fanfilcon, somofilcon, samfilcon, olifilcon, asmofilcon, formofilcon, stenfilcon, abafilcon, mangofilcon, riofilcon, sifilcon, larafilcon, and delefilcon. In particular, from the viewpoint of exhibiting good water wettability and lubricity, lotrafilcon, galyfilcon, narafilcon, senofilcon, comfilcon, enfilcon, stenfilcon, somofilcon, delefilcon, balafilcon, and samfilcon are more preferred, lotrafilcon, narafilcon, senofilcon, comfilcon, and enfilcon are still more preferred, and narafilcon, senofilcon, and comfilcon are particularly preferred.

The low water content soft material and the low water content hard material are preferably materials containing a silicon atom. This is because when the materials are used in, for example, a medical device such as an ophthalmic lens, the materials exhibit high oxygen permeability to enable sufficient oxygen supply to the cornea.

For example, when the low water content hard material is contained in a contact lens, preferred specific examples of the low water content hard material include low water content hard materials selected from the group belonging to the classes of contact lens materials defined by the U.S. Food and Drug Administration (FDA).

The low water content hard material is preferably a polymer containing a silicon atom in the main chain and/or the side chain. Examples thereof include a polymer containing a siloxane bond. Among these polymers containing a silicon atom, those in which the silicon atom is contained in the polymer by a siloxane bond are preferred from the viewpoint of oxygen permeability. Specific examples of such polymers include homopolymers containing tris(trimethylsiloxy)silylpropyl methacrylate, polydimethylsiloxane having double bonds at both ends, silicone-containing (meth)acrylate or the like, and copolymers of these monomers with other monomers.

Specifically, the low water content hard material is preferably a material selected from the group consisting of neofocon, pasifocon, telefocon, silafocon, paflufocon, petrafocon, and fluorofocon. In particular, from the viewpoint of exhibiting good water wettability and antifouling properties, neofocon, pasifocon, telefocon, and silafocon are more preferred, neofocon, pasifocon, and telefocon are still more preferred, and neofocon is particularly preferred.

In the present invention, in an aspect in which the medical device is other than a contact lens, suitable examples of the low water content hard material include polyethylene, polypropylene, polysulfone, polyetherimide, polystyrene, polymethyl methacrylate, polyamide, polyester, epoxy resins, polyurethane, and polyvinyl chloride. In particular, from the viewpoint of exhibiting good water wettability and antifouling properties, the low water content hard material is more preferably polysulfone, polystyrene, polymethyl methacrylate, or polyamide, and is particularly preferably polymethyl methacrylate.

Specific examples of the low water content soft material include a low water content soft material as described in International Publication No. 2013/024799. The low water content soft material has a water content of 10 mass% or less, an elastic modulus of 100 kPa or more and 2,000 kPa or less, and a tensile elongation of 50% or more and 3,000% or less, and is used in a medical device. Elastofilcon is also suitable.

In the present invention, in an aspect in which the medical device is other than an ophthalmic lens including a contact lens, suitable examples of the low water content soft material include silicone elastomers, soft polyurethane, polyvinyl acetate, ethylene-vinyl acetate copolymers, soft polyester resins, soft acrylic resins, soft polyvinyl chloride, natural rubbers, and various synthetic rubbers.

According to the present invention, it is possible to impart moderate hydrophilicity (water wettability) to the surface of the medical device regardless of whether the substrate is a water-containing substrate or a low water content substrate. Therefore, the water content of the substrate may have any value of 0 to 99 mass%. The water content of the substrate is preferably 0.0001 mass% or more, and particularly preferably 0.001 mass% or more, because a higher effect of imparting moderate hydrophilicity to the surface of the medical device is exerted. The water content of the substrate is preferably 60 mass% or less, more preferably 50 mass% or less, and still more preferably 40 mass% or less.

When the medical device is a contact lens, the water content of the substrate is preferably 15 mass% or more, and more preferably 20 mass% or more, because movement of the lens in the eye is easily secured.

The hydrophilic polymer used in the method for manufacturing a medical device of the present invention is usually a material different from that of the substrate. However, the material may be the same as the material that constitutes the substrate as long as a predetermined effect is obtained.

The hydrophilic polymer is composed of a hydrophilic material. However, the hydrophilic polymer may contain other additives and the like as long as the exhibition of hydrophilicity is not impaired. Here, the hydrophilic material is a material soluble in 100 parts by mass of water or a mixed liquid of 100 parts by mass of water and 100 parts by mass of tert-butanol at room temperature (20 to 23°C) in an amount of 0.0001 parts by mass or more, more preferably 0.01 parts by mass or more, still more preferably 0.1 parts by mass or more, and particularly preferably 1 part by mass or more.

In the method for manufacturing a medical device of the present invention, (a) the hydrophilic polymer is a (co) polymer of a (meth) acrylamide having two substituents each having 2 or more carbon atoms on a nitrogen atom. In the present invention, the "(co)polymer of a (meth) acrylamide having two substituents each having 2 or more carbon atoms on a nitrogen atom" refers to a (co)polymer containing, as a (co)polymerization component, a (meth) acrylamide having two substituents each having 2 or more carbon atoms on a nitrogen atom. Such a hydrophilic polymer is preferred because a

surface having excellent and durable water wettability can be formed.

The substituent having 2 or more carbon atoms may be linear or branched, and is preferably an alkyl group having 2 to 6 carbon atoms from the viewpoint of being capable of imparting good and durable hydrophilicity to the medical device. Examples of the substituent include an ethyl group, a 1-propyl group, a 2-propyl group, a 1-butyl group, a 2-butyl group, a tert-butyl group, a 1-pentyl group, a 2-pentyl group, a 3-pentyl group, and a hexyl group. In the present specification, any notation of "alkyl group" without a position number encompasses all alkyl groups having any position number of the alkyl group (for example, a "hexyl group" encompasses all of a "1-hexyl group", a "2-hexyl group", and the like). Among them, an ethyl group, a 1-propyl group, and a 2-propyl group are more preferred, and an ethyl group is particularly preferred.

As for the combination of two substituents in the wording "having two substituents", a combination of the same substituents or a combination of different substituents can be suitably used. Specific examples of the (meth)acrylamide having two substituents each having 2 or more carbon atoms on a nitrogen atom include N,N-diethyl (meth)acrylamide, N-ethyl-N-propyl (meth)acrylamide, and N-butyl-N-ethyl (meth)acrylamide. Among them, N,N-diethyl (meth)acrylamide is particularly preferred because it can impart good hydrophilicity to the medical device.

The (meth) acrylamide may be used singly or in combination of two or more types thereof.

In addition, the (co)polymer of the (meth)acrylamide having two substituents each having 2 or more carbon atoms on a nitrogen atom can also contain, as a copolymerization component, one type or a plurality of types of different monomers having an amide group.

When the (co)polymer contains, as a copolymerization component, the different monomer having an amide group, the hydrophilic polymer exhibits moderate viscosity upon being dissolved in water, so that a surface having not only water wettability but also lubricity can be formed. In the present invention, the amide group is a group having a structure represented by N-C=O.

The different monomer having an amide group is preferably a monomer selected from monomers having a (meth) acrylamide group and N-vinyl carboxylic acid amides (including cyclic amides) from the viewpoint of ease of polymerization.

Suitable examples of such monomers include N-vinylpyrrolidone, N-vinylcaprolactam, N-vinylacetamide, N-methyl-N-vinylacetamide, N-vinylformamide, N,N-dimethyl (meth)acrylamide, N-isopropyl (meth)acrylamide, N-methyl (meth)acrylamide, N-ethyl (meth)acrylamide, N-butyl (meth)acrylamide, N-tert-butyl (meth)acrylamide, N-hydroxymethyl acrylamide, N-methoxymethyl acrylamide, N-ethoxymethyl acrylamide, N-propoxymethyl acrylamide, N-isopropoxymethyl acrylamide, N-(2-hydroxyethyl) (meth)acrylamide, N-butoxymethyl acrylamide, N-isobutoxymethyl acrylamide, N-hydroxymethyl methacrylamide, N-methoxymethyl methacrylamide, N-ethoxymethyl methacrylamide, N-propoxymethyl methacrylamide, N-butoxymethyl methacrylamide, N-isobutoxymethyl methacrylamide, acryloylmorpholine, and acrylamide. Among them, N-vinylpyrrolidone, N-isopropyl (meth)acrylamide, and N,N-dimethyl (meth) acrylamide are preferred from the viewpoint of lubricity, N-isopropyl (meth)acrylamide and N,N-dimethyl (meth)acrylamide are more preferred, and N,N-dimethyl (meth)acrylamide is particularly preferred.

When a copolymer of the (meth)acrylamide having two substituents each having 2 or more carbon atoms on a nitrogen atom with the different monomer having an amide group is used, the copolymer preferably has a copolymerization ratio of [mass of the different monomer having an amide group]/[mass of the (meth)acrylamide monomer having two substituents each having 2 or more carbon atoms on a nitrogen atom] of 1/99 to 99/1. The copolymerization ratio of the different monomer having an amide group is more preferably 2 mass% or more, still more preferably 5 mass% or more, even more preferably 7 mass% or more, and still even more preferably 10 mass% or more. The copolymerization ratio of the different monomer having an amide group is more preferably 90 mass% or less, still more preferably 80 mass% or less, and even more preferably 70 mass% or less. The copolymerization ratio of the (meth)acrylamide monomer having two substituents each having 2 or more carbon atoms on a nitrogen atom is more preferably 10 mass% or more, still more preferably 20 mass% or more, and even more preferably 30 mass% or more. The copolymerization ratio of the (meth) acrylamide monomer having two substituents each having 2 or more carbon atoms on a nitrogen atom is more preferably 98 mass% or less, still more preferably 95 mass% or less, even more preferably 93 mass% or less, and still even more preferably 90 mass% or less. When the copolymerization ratio of the (meth)acrylamide monomer having two substituents each having 2 or more carbon atoms on a nitrogen atom to the different monomer having an amide group is within the above-mentioned range, functions such as water wettability and lubricity are easily exhibited.

In the method for manufacturing a medical device of the present invention, the hydrophilic polymer is preferably a copolymer of the (meth) acrylamide having two substituents each having 2 or more carbon atoms on a nitrogen atom with (meth)acrylic acid. Such a hydrophilic polymer is preferred because an excellent surface having durability, which is excellent not only in water wettability but also in antifouling properties against body fluids and the like can be formed.

Preferred specific examples of the copolymer of the (meth) acrylamide having two substituents each having 2 or more carbon atoms on a nitrogen atom with (meth) acrylic acid include a (meth)acrylic acid/N,N-diethylacrylamide copolymer, a (meth)acrylic acid/N-ethyl-N-propyl (meth)acrylamide copolymer, and a (meth)acrylic acid/N-butyl-N-ethyl (meth) acrylamide copolymer. A (meth) acrylic acid/N,N-diethyl (meth)acrylamide copolymer is particularly preferred.

When the copolymer of the (meth)acrylamide having two substituents each having 2 or more carbon atoms on a nitrogen atom with (meth) acrylic acid is used, the copolymer preferably has a copolymerization ratio of [mass of the (meth) acrylic acid monomer]/[mass of the (meth)acrylamide monomer having two substituents each having 2 or more carbon atoms on a nitrogen atom] of 1/99 to 99/1. The copolymerization ratio of the (meth) acrylic acid monomer is more preferably 2 mass% or more, still more preferably 5 mass% or more, even more preferably 7 mass% or more, and still even more preferably 10 mass% or more. The copolymerization ratio of the (meth)acrylic acid monomer is more preferably 90 mass% or less, still more preferably 80 mass% or less, and even more preferably each of 70 mass% or less, 60 mass% or less, 50 mass% or less, 40 mass% or less, and 30 mass% or less. The copolymerization ratio of the (meth)acrylamide monomer having two substituents each having 2 or more carbon atoms on a nitrogen atom is more preferably 10 mass% or more, still more preferably 20 mass% or more, and even more preferably each of 30 mass% or more, 40 mass% or more, 50 mass% or more, 60 mass% or more, and 70 mass% or more. The copolymerization ratio of the (meth)acrylamide monomer having two substituents each having 2 or more carbon atoms on a nitrogen atom is more preferably 98 mass% or less, still more preferably 95 mass% or less, even more preferably 93 mass% or less, and still even more preferably 90 mass% or less. When the copolymerization ratio of the (meth)acrylamide monomer having two substituents each having 2 or more carbon atoms on a nitrogen atom to the (meth)acrylic acid monomer is within the above-mentioned range, functions such as water wettability and antifouling properties against body fluids are easily exhibited.

In addition, the (co)polymer of the (meth)acrylamide having two substituents each having 2 or more carbon atoms on a nitrogen atom can further contain, as a copolymerization component, one type or a plurality of types of monomers other than (meth) acrylic acid and having no amide group. Hereinafter, the "monomer other than (meth) acrylic acid and having no amide group" may be referred to as a monomer X.

Suitable examples of the monomer X include hydroxyethyl (meth)acrylate, hydroxypropyl (meth)acrylate, hydroxybutyl (meth)acrylate, glycerol (meth)acrylate, caprolactone-modified 2-hydroxyethyl (meth)acrylate, N-(4-hydroxyphenyl) maleimide, hydroxystyrene, and vinyl alcohol (carboxylic acid vinyl ester as a precursor). Among them, a monomer having a (meth) acryloyl group is preferred from the viewpoint of ease of polymerization, and a (meth)acrylic acid ester monomer is more preferred. From the viewpoint of improving the antifouling properties against body fluids, hydroxyethyl (meth)acrylate, hydroxypropyl (meth)acrylate, and glycerol (meth)acrylate are preferred, and hydroxyethyl (meth)acrylate is particularly preferred.

It is also possible to use, as the monomer X, a monomer that exhibits functions such as hydrophilicity, antibacterial properties, antifouling properties, and drug efficacy.

Specific examples of the monomer having antibacterial properties include a monomer having a quaternary ammonium salt. Examples of such monomers include monomers having antibacterial properties, such as imidazolium salt monomers described in Japanese Unexamined Patent Application Publication No. 2010-88858, (3-acrylamidopropyl) trimethylammonium chloride, trimethyl-2-methacryloyloxyethyl ammonium chloride, and 2-(meth)acryloyloxyethyl phosphorylcholine.

When the (co)polymer of the (meth) acrylamide having two substituents each having 2 or more carbon atoms on a nitrogen atom contains the monomer X as a copolymerization component, the copolymerization ratio of the monomer X is preferably 2 mass% or more, more preferably 5 mass% or more, and still more preferably 10 mass% or more. The copolymerization ratio of the monomer X is preferably 90 mass% or less, more preferably 80 mass% or less, and still more preferably 70 mass% or less.

When the copolymerization ratio of the (meth)acrylamide having two substituents each having 2 or more carbon atoms on a nitrogen atom to the monomer X in the (co)polymer is within the above-mentioned range, functions such as water wettability, antifouling properties against body fluids, and antibacterial properties are easily exhibited.

In addition, the aqueous packaging solution may contain additives and the like other than the above-mentioned materials as long as the properties required of the medical device are not impaired. Furthermore, the aqueous packaging solution may contain, in addition to the (co)polymer of the (meth) acrylamide having two substituents each having 2 or more carbon atoms on a nitrogen atom, one type or a plurality of other hydrophilic polymers. However, from the viewpoint of an increase in the manufacturing cost, the hydrophilic polymer is preferably composed only of one type of (co)polymer of the (meth) acrylamide having two substituents each having 2 or more carbon atoms on a nitrogen atom.

Here, one type of polymer means a polymer or a polymer group (isomers, complexes, and the like) manufactured by one synthesis reaction. When a plurality of monomers are used to form each copolymer, even if the copolymers contain the same constituent monomer species, the polymers synthesized at varied blend ratios are not regarded as those of the same type.

In addition, the wording "the hydrophilic polymer is composed only of one type of (co)polymer of the (meth) acrylamide having two substituents each having 2 or more carbon atoms on a nitrogen atom" means that the hydrophilic polymer does not contain any polymer different from the hydrophilic polymer, or even if the hydrophilic polymer contains such a different polymer, the content of the different polymer is preferably 3 parts by mass or less with respect to 100 parts by mass of the hydrophilic polymer. The content of the different polymer is more preferably 0.1 parts by mass or less, and still more preferably 0.0001 parts by mass or less.

When the different polymer contains basic groups, the number ratio of basic groups/acidic groups in all the polymers contained in the aqueous packaging solution is preferably 0.20 or less. The ratio is more preferably 0.10 or less, and still more preferably 0.05 or less because a salt derived from a reaction between the acidic groups and the basic groups is not formed, and the aqueous packaging solution has excellent transparency. Here, the acidic group refers to an acidic functional group, and examples thereof include a group selected from a carboxyl group and a sulfonic acid group, and a salt thereof. The basic group refers to a basic functional group, and examples thereof include an amino group and a salt thereof.

Next, a method for manufacturing the medical device of the present invention will be described. In the present invention, the medical device can be obtained by a method including a step of heating an aqueous packaging solution, wherein the heating step is performed in a state in which the aqueous packaging solution contains at least one type of hydrophilic polymer, and a substrate of the medical device is at least partially in contact with the aqueous packaging solution, and the method satisfies all of specific requirements. Here, the present inventors have found that it is possible by a very simple method to impart, to the medical device, highly durable hydrophilicity, that is, water wettability, which cannot be achieved by the prior art. In this method, the hydrophilic polymer is a (co)polymer of the (meth)acrylamide having two substituents each having 2 or more carbon atoms on a nitrogen atom, and the aqueous packaging solution, which has a mass% concentration of the hydrophilic polymer contained therein in the range of 0.0001 to 30 mass%, is heated in a state in which the medical device is at least partially in contact with the aqueous packaging solution. In addition, the applicable substrate is not limited to a water-containing hydrogel and a silicone hydrogel. Furthermore, since the aqueous packaging solution has a pH in the range of 6.1 to 8.0 after the heating step, particularly when the heating step is a sterilization step, it is possible to impart highly durable water wettability, lubricity, and the like to the medical device simultaneously with the sterilization step without increasing the number of manufacturing steps. This matter has an industrially very important meaning in that no additional manufacturing steps are required. Therefore, in the method for manufacturing a medical device of the present invention, the heating step is preferably a sterilization step.

Next, the molecular weight and concentration of the hydrophilic polymer will be described. In the prior art, there is a problem that it is difficult to impart sufficient water wettability or durable water wettability to a device when the surface of the substrate is modified simultaneously with sterilization using only one type of hydrophilic polymer. In the present invention, however, it is easy to impart good and durable water wettability and lubricity to the substrate even when the surface of the substrate is modified simultaneously with sterilization using only one type of hydrophilic polymer. This is because the hydrophilic polymer has high adsorption power to the substrate.

The hydrophilic polymer used in the present invention preferably has a molecular weight of 2,000 to 1,500,000. The molecular weight is more preferably 5,000 or more, and still more preferably 10,000 or more. The molecular weight is more preferably 1,200,000 or less, and still more preferably 1,000,000 or less. Here, the mass average molecular weight in terms of polyethylene glycol measured by a gel permeation chromatography method (aqueous solvent) is used as the molecular weight.

If the concentration of the hydrophilic polymer in the aqueous packaging solution during the manufacture is too high, handling difficulty during the manufacture may increase due to an increase in viscosity. Therefore, in the method for manufacturing a medical device of the present invention, (b) the aqueous packaging solution has a mass% concentration of the hydrophilic polymer in the range of 0.0001 to 30 mass%. The concentration of the hydrophilic polymer is more preferably 0.001 mass% or more, and still more preferably 0.005 mass% or more. The concentration of the hydrophilic polymer is more preferably 10 mass% or less, still more preferably 5 mass% or less, more preferably 1 mass%, and most preferably 0.1 mass% or less.

As for the heating step, since the medical device can be used as it is without being additionally subjected to a washing step after the heating, (c) the aqueous packaging solution has a pH in the range of 6.1 to 8.0 after the heating step. The pH is more preferably 6.5 or more, more preferably 6.6 or more, still more preferably 6.7 or more, and even more preferably 6.8 or more. The pH is preferably 7.9 or less, more preferably 7.8 or less, and still more preferably 7.6 or less. A pH of less than 6.1 or more than 8.0 is not preferred because in such a case, in the case of using the aqueous packaging solution in, for example, a medical device such as an ophthalmic lens, it is necessary to additionally perform a washing step and a sterilization step in a neutral solution in order to eliminate irritation to the eye, and the manufacturing steps are complicated.

The pH of the aqueous packaging solution may slightly change during the heating operation, but the pH before the heating operation is also preferably in the range of 6.1 to 8.0 as described above.

The pH of the aqueous packaging solution can be measured using a pH meter (for example, pH meter Eutech pH 2700 (Eutech Instruments)). Here, the pH of the aqueous packaging solution before heating refers to a value of pH measured after all the hydrophilic polymers are added to the solution, and the solution is stirred at room temperature (20 to 23°C) for 30 minutes using a rotor so as to be uniform. In the present invention, the pH value is rounded off to the first decimal place.

Preferred examples of the solvent of the aqueous packaging solution containing the hydrophilic polymer include a water-soluble organic solvent, water, and a mixed solvent thereof. A mixed solvent of water and a water-soluble organic

solvent, and water are more preferred, and water is most preferred. As for the water-soluble organic solvent, various water-soluble alcohols are suitable, water-soluble alcohols having 6 or less carbon atoms are more suitable, and water-soluble alcohols having 5 or less carbon atoms are still more suitable.

It is also preferred to further add a buffer to these solvents.

As for the buffer, any physiologically compatible known buffer can be used. Buffers are known to persons skilled in the art, and examples thereof include the following compounds: boric acid, borates (e.g. sodium borate), citric acid, citrates (e.g. potassium citrate), bicarbonates (e.g. sodium bicarbonate), phosphate buffer solutions (e.g. $Na_2HPO_4$, $NaH_2PO_4$, and $KH_2PO_4$), TRIS (tris(hydroxymethyl)aminomethane), 2-bis(2-hydroxyethyl)amino-2-(hydroxymethyl)-1,3-propanedio l, bis-aminopolyol, triethanolamine, ACES (N-(2-acetamide)-2-aminoethanesulfonic acid), BES (N,N-bis(2-hydroxyethyl)-2-aminoethanesulfonic acid), HEPES (4-(2-hydroxyethyl)-1-piperazineethanesulfonic acid), MES (2-(N-morpholino)ethanesulfonic acid), MOPS (3-[N-morpholino]-propanesulfonic acid), PIPES (piperazine-N,N'-bis(2-ethanesulfonic acid)), TES (N-[tris(hydroxymethyl)methyl]-2-aminoethanesulfonic acid), and salts thereof.

The amount of each buffer used may be an amount necessary for being effective for achieving a desired pH. Usually, the amount of the buffer is preferably 0.001 mass% to 2 mass% in the solution. The amount of the buffer is more preferably 0.01 mass% or more, and still more preferably 0.05 mass% or more. The amount of the buffer is more preferably 1 mass% or less, and still more preferably 0.30 mass% or less.

The pH of the buffer solution before the addition of the hydrophilic polymer is preferably 6.3 to 7.8, which is a physiologically acceptable range. The pH of the buffer solution is preferably 6.5 or more, and more preferably 6.8 or more. The pH of the buffer solution is preferably 7.6 or less, and more preferably 7.4 or less.

The heating temperature is preferably 100°C to 200°C from the viewpoint of obtaining a medical device surface that exhibits good and durable water wettability and lubricity, and of giving little influence on the strength of the medical device itself. The heating temperature is more preferably 105°C or more, still more preferably 110°C or more, even more preferably 115°C or more, and most preferably 121°C or more. The heating temperature is more preferably 180°C or less, still more preferably 170°C or less, and most preferably 150°C or less.

The heating time is preferably 5 minutes to 600 minutes. This is because if the heating time is too short, a medical device surface that exhibits good water wettability and lubricity is not obtained, and if the heating time is too long, the strength of the medical device itself may be affected. The heating time is more preferably 10 minutes or more, and still more preferably 15 minutes or more. The heating time is more preferably 400 minutes or less, and still more preferably 300 minutes or less. The heating time in the present invention refers to a time from the time point at which the temperature reaches the heating temperature to the time point at which the heating is stopped.

Examples of the method for sterilization in the method for manufacturing a medical device of the present invention include a high-pressure steam sterilization method, a dry heat sterilization method, a flame sterilization method, a boiling disinfection method, a free-flowing steam disinfection method, an ethylene oxide gas sterilization method (EOG sterilization method), radiation sterilization (a gamma ray sterilization method), and an ultraviolet sterilization method. The high-pressure steam sterilization method is most preferred from the viewpoint of being capable of imparting good and highly durable water wettability and lubricity to the substrate, and of the manufacturing cost. The sterilizer used is preferably an autoclave. That is, in the method for manufacturing a medical device of the present invention, the heating is preferably performed by autoclave sterilization (high-pressure steam sterilization using an autoclave).

After the sterilization treatment, the obtained medical device may be further subjected to other treatments. Examples of such other treatments include a method in which the medical device is subjected to a similar sterilization treatment again in a solution containing a hydrophilic polymer; a method in which the aqueous packaging solution is replaced with another aqueous packaging solution not containing a hydrophilic polymer, and the medical device is subjected to a similar sterilization treatment; a method in which radiation irradiation is performed; a method in which an LbL treatment (Layer by Layer treatment) is performed, which is a treatment of alternately applying polymer materials having opposite charges layer by layer; a method in which the medical device is subjected to a crosslinking treatment using a metal ion; and a method in which the medical device is subjected to a chemical crosslinking treatment.

The substrate may be pretreated before the sterilization treatment. Examples of the pretreatment include a hydrolysis treatment with an acid such as polyacrylic acid or an alkali such as sodium hydroxide.

However, in light of the idea of the present invention that it is possible to impart durable hydrophilicity to the substrate surface by a simple method, it is preferred to perform such treatments without excessively complicating the manufacturing steps.

As for the radiation used in the radiation irradiation, various ion beams, electron beams, positron beams, X-rays, $\gamma$-rays, and neutron beams are preferred, electron beams and $\gamma$-rays are more preferred, and $\gamma$-rays are most preferred.

As for the LbL treatment, for example, a treatment as described in International Publication No. 2013/024800 is preferably employed, in which an acidic polymer and a basic polymer are used.

As for the metal ion used in the crosslinking treatment using a metal ion, various metal ions are preferred, monovalent and divalent metal ions are more preferred, and divalent metal ions are most preferred. A chelate complex may also be used.

As for the chemical crosslinking treatment, for example, it is possible to employ a reaction between an epoxy group and a carboxyl group as described in Japanese Unexamined Patent Application Publication No. 2014-533381, or a crosslinking treatment of forming a crosslink with a known acidic hydrophilic polymer having a hydroxyl group.

In the above-mentioned method in which the solution is replaced with a solution not containing a hydrophilic polymer and the medical device is subjected to a similar sterilization treatment, the solution not containing a hydrophilic polymer is not particularly limited, but is preferably a buffer solution. As for the buffer, those described above can be used.

The pH of the buffer solution is preferably 6.3 to 7.8, which is a physiologically acceptable range. The pH of the buffer solution is preferably 6.5 or more, and more preferably 6.8 or more. The pH of the buffer solution is preferably 7.6 or less, and more preferably 7.4 or less.

In addition, in the method for manufacturing a medical device of the present invention, the medical device preferably includes a hydrophilic polymer layer on at least a part of the substrate, because the hydrophilic polymer can impart good and highly durable water wettability to the substrate. Although it depends on the application, including the hydrophilic polymer layer on at least a part of the substrate means, for example, that the polymer layer is present on the entire one surface of the substrate. When the substrate does not have a thickness or has a two-dimensional shape with an ignorable thickness, it is preferred that the polymer layer be present on the entire one surface of the substrate. It is also preferred that the polymer layer be present on the entire surface of the substrate.

In addition, it is preferred that the hydrophilic polymer do not have a covalent bond with the substrate because in such a case, the medical device can be manufactured in a simple process regardless of whether the substrate is water-containing or water-free. The absence of a covalent bond is determined by the fact that the medical device does not include a chemically reactive group or a group generated by a reaction of a chemically reactive group. Specific examples of the chemically reactive group include, but are not limited to, an azetidinium group, an epoxy group, an isocyanate group, an aziridine group, an azlactone group, and combinations thereof.

The thickness of the hydrophilic polymer layer is preferably 1 nm or more and less than 100 nm as determined in the observation of a vertical cross section of the device in a dry state using a transmission electron microscope. When the thickness is within the above-mentioned range, functions such as water wettability and lubricity are easily exhibited. The thickness is more preferably 5 nm or more, and still more preferably 10 nm or more. The thickness is more preferably 95 nm or less, still more preferably 90 nm or less, still more preferably 85 nm or less, still more preferably 50 nm or less, still more preferably 30 nm or less, still more preferably 20 nm or less, still more preferably 15 nm or less, and particularly preferably 10 nm or less. When the thickness of the hydrophilic polymer layer is less than 100 nm, excellent water wettability and lubricity are exhibited. For example, when the hydrophilic polymer layer is used in a medical device such as an ophthalmic lens, refraction of light for focusing on the retina is not disturbed, and poor visibility is less likely to occur.

In addition, in the method for manufacturing a medical device of the present invention, the hydrophilic polymer layer is preferably present in the medical device in a state of being at least partially mixed with the substrate. The state in which the hydrophilic polymer layer is mixed with the substrate is confirmed in the observation of the cross section of the medical device using an observation means that allows elemental analysis or composition analysis, such as scanning transmission electron microscopy, electron energy loss spectroscopy, energy dispersive X-ray spectroscopy, or time-of-flight secondary ion mass spectrometry. The matter can be confirmed by the fact that an element derived from the substrate is detected in the cross-sectional structure of the substrate before and after formation of the hydrophilic polymer layer, and at least a part of the hydrophilic polymer layer. When the hydrophilic polymer layer is mixed with the substrate, the hydrophilic polymer can be fixed to the substrate more firmly.

When the hydrophilic polymer layer is present in a state of being at least partially mixed with the substrate, it is preferred that a two-layer structure be observed, the structure being composed of a "layer in which the hydrophilic polymer layer is at least partially mixed with the substrate" (hereinafter referred to as a mixed layer) and a "layer made of the hydrophilic polymer" (hereinafter referred to as a single component layer). The thickness of the mixed layer is preferably 3% or more, more preferably 5% or more, and still more preferably 10% or more with respect to the total thickness of the mixed layer and the single component layer. The thickness of the mixed layer is preferably 98% or less, more preferably 95% or less, still more preferably 90% or less, and particularly preferably 80% or less with respect to the total thickness of the mixed layer and the single component layer. A percentage of thickness of the mixed layer of 3% or more is preferred because the hydrophilic polymer and the substrate are sufficiently mixed, so that the hydrophilic polymer can be fixed to the substrate more firmly. A percentage of thickness of the mixed layer of 98% or less is preferred because the hydrophilicity of the hydrophilic polymer is easily exhibited sufficiently.

In the method for manufacturing a medical device of the present invention, when the medical device is, for example, a medical device used in a state of being attached to a living body surface, or an ophthalmic device such as an ophthalmic lens, it is preferred that the surface of the medical device have a long liquid film retention time. This is from the viewpoint of preventing the medical device from sticking to the skin or the like of the user, and of preventing the medical device from sticking to the cornea of the wearer.

Here, the liquid film retention time in the present invention refers to a time during which the liquid film on the surface of the medical device is retained, which is measured by immersing and leaving the medical device to stand in a

phosphate buffer solution, and then pulling up the medical device from the phosphate buffer solution and retaining the medical device in the air. Specifically, the liquid film retention time is a time determined by pulling up the medical device that has been immersed and left to stand in the phosphate buffer solution from the liquid, and retaining the medical device in the air so that the surface may be vertical. The liquid film retention time is a time during which the liquid film on the surface of the medical device is retained without being broken. Note that "the liquid film is broken" refers to a state in which a phenomenon of repelling water occurs on the surface of the medical device.

In the method for manufacturing a medical device of the present invention, the time during which the liquid film on the surface of the medical device is retained (liquid film retention time) is preferably 10 seconds or more. The liquid film retention time is measured by immersing and leaving the medical device to stand in a phosphate buffer solution, and then pulling up the medical device from the phosphate buffer solution and retaining the medical device in the air. The liquid film retention time is more preferably 15 seconds or more, and still more preferably 20 seconds or more. The upper limit of the range of the liquid film retention time is not particularly limited, but it is preferably 300 seconds or less, and more preferably 200 seconds or less. This is because if the liquid film retention time is too long, moisture evaporation from the surface of the medical device easily proceeds, and the effect of the hydrophilic polymer layer is reduced.

In the prior art, even in a medical device having good water wettability, the water wettability is extremely reduced after scrubbing and washing, and the once reduced water wettability tends not to recover only by leaving the medical device in water at room temperature for a short time (for example, about 2 hours). That is, reduction in water wettability has been observed, which is considered to be caused by peeling off or elution of the hydrophilic polymer on the substrate surface due to scrubbing and washing. Therefore, a medical device whose water wettability is reduced after scrubbing and washing is not preferred because the medical device may be changed in the surface state by an external stimulus and reduced in water wettability. Conversely, it can be said that a medical device in which water wettability of the surface is not reduced even after scrubbing and washing, or a medical device in which water wettability of the surface, even if being reduced, recovers in a short time, is an excellent medical device in which the surface state is hardly changed by an external stimulus.

In the method for manufacturing a medical device of the present invention, when the medical device is an ophthalmic device such as an ophthalmic lens, it is preferred that the surface of the medical device have a long liquid film retention time after scrubbing and washing from the viewpoint of reducing the feeling of dryness and maintaining good use comfort for a long time.

Specifically, the medical device is scrubbed and washed 50 times with human fingers and then immersed in a phosphate buffer solution at room temperature for 2 hours, and then the liquid film retention time of the surface of the medical device is evaluated. When the liquid film retention time of the surface of the medical device after 2 hours is 10 seconds or more, it means that the surface of the medical device has sufficient water wettability and durability. The liquid film retention time is preferably 10 seconds or more, more preferably 15 seconds or more, and particularly preferably 20 seconds or more. In particular, it is preferred that the medical device have a liquid film retention time comparable to that before scrubbing and washing, because in such a case, the medical device exhibits more excellent durability. Details of the measurement method will be described later.

In addition, in the method for manufacturing a medical device of the present invention, when the medical device is, for example, a medical device used by being inserted into a living body, the surface of the medical device preferably has excellent lubricity. As for an index of lubricity, it is preferred that the friction coefficient measured by the method described in the section of EXAMPLES in the present specification be small. The friction coefficient is preferably 0.700 or less, more preferably 0.500 or less, and particularly preferably 0.300 or less. Alternatively, if the friction is extremely small, handling at the time of putting in and out the medical device tends to be difficult. Thus, the friction coefficient is preferably 0.001 or more, and more preferably 0.002 or more.

In the method for manufacturing a medical device of the present invention, the tensile elastic modulus of the medical device is appropriately selected according to the type of the medical device. In the case of a soft medical device such as an ophthalmic lens, the tensile elastic modulus is preferably 10 MPa or less, preferably 5 MPa or less, more preferably 3 MPa or less, still more preferably 2 MPa or less, even more preferably 1 MPa or less, and most preferably 0.6 MPa or less. The tensile elastic modulus is preferably 0.01 MPa or more, more preferably 0.1 MPa or more, still more preferably 0.2 MPa or more, and most preferably 0.25 MPa or more. In the case of a soft medical device such as an ophthalmic lens, if the tensile elastic modulus is too small, the soft medical device is too soft and tends to be difficult to handle. If the tensile elastic modulus is too large, the soft medical device is too hard, and use comfort and wearing comfort tend to deteriorate.

In the method for manufacturing a medical device of the present invention, the tensile elastic modulus change rate of the substrate before and after the heating step is preferably 15% or less, more preferably 14% or less, and particularly preferably 13% or less. Too large a tensile elastic modulus change rate is not preferred because deformation and poor feeling of use may be caused. Details of the measurement method will be described later.

In the method for manufacturing a medical device of the present invention, the antifouling properties of the medical device can be evaluated by lipid (methyl palmitate) adhesion. A smaller amount of adhesion according to the

evaluation is preferred because the medical device provides more excellent feeling of use, and the risk of bacterial propagation is reduced. Details of the measurement method will be described later.

In the method for manufacturing a medical device of the present invention, the amount of change of the water content between the medical device obtained after completion of the heating step and the substrate before the heating step is preferably 10 percentage points or less. Here, the amount of change (percentage point) of the water content is a difference between the water content (mass%) of the obtained medical device and the water content (mass%) of the substrate as a raw material of the medical device.

In the case where the medical device is used as an ophthalmic device such as an ophthalmic lens, the amount of change of the water content of the substrate before and after the heating step is preferably 10 percentage points or less, more preferably 8 percentage points or less, and particularly preferably 6 percentage points or less from the viewpoint of preventing poor visibility and deformation caused by distortion of the refractive index due to improvement in the water content. Details of the measurement method will be described later.

In addition, in the case where the medical device is used as an ophthalmic device such as an ophthalmic lens, the size change rate of the substrate before and after the heating step is preferably 5% or less, more preferably 4% or less, and particularly preferably 3% or less from the viewpoint of preventing corneal damage associated with deformation. Details of the measurement method will be described later.

EXAMPLES

Hereinafter, the present invention will be specifically described with reference to examples, but the present invention is not limited to these examples. First, analysis methods and evaluation methods will be described.

<Water wettability (liquid film retention time)>

To eliminate the influence of the hydrophilic polymer contained in the aqueous packaging solution and not adsorbed to the medical device, the medical device was left to stand at room temperature for 1 hour or more in 3 mL of a phosphate buffer solution in a glass vial. For the evaluation of only the commercially available contact lenses described in the comparative examples, to eliminate the influence of the hydrophilic polymer contained in the packaging solution and not adsorbed to the lens, the lens was left to stand at room temperature for 1 hour or more in 3 mL of a phosphate buffer solution in a glass vial.

The medical device was pulled up from the phosphate buffer solution in which the medical device had been immersed and left to stand, and was retained in the air. The time during which the liquid film on the surface was retained was visually observed, and the average of N = 3 was judged according to the following criteria.

A: The liquid film on the surface is retained for 20 seconds or more.
B: The liquid film on the surface is broken within 15 seconds or more and less than 20 seconds.
C: The liquid film on the surface is broken within 10 seconds or more and less than 15 seconds.
D: The liquid film on the surface is broken within 1 second or more and less than 10 seconds.
E: The liquid film on the surface is instantaneously broken (within less than 1 second).

<Water wettability 2 hours after scrubbing and washing (liquid film retention time)>

To eliminate the influence of the hydrophilic polymer contained in the aqueous packaging solution and not adsorbed to the medical device, the medical device was left to stand at room temperature for 1 hour in 3 mL of a phosphate buffer solution in a glass vial. For the evaluation of only the commercially available contact lenses described in the comparative examples, to eliminate the influence of the hydrophilic polymer contained in the packaging solution and not adsorbed to the lens, the lens was left to stand at room temperature for 1 hour in 3 mL of a phosphate buffer solution in a glass vial.

Then, the lens was sandwiched between the thumb and the forefinger, and scrubbed and washed 50 times. The lens was then returned to the phosphate buffer solution in which the lens had been immersed and left to stand, and left to stand for 2 hours.

The medical device was pulled up from the phosphate buffer solution in which the medical device had been immersed and left to stand, and was retained in the air. The time during which the liquid film on the surface was retained was visually observed, and the average of N = 3 was judged according to the following criteria.

A: The liquid film on the surface is retained for 20 seconds or more.
B: The liquid film on the surface is broken within 15 seconds or more and less than 20 seconds.
C: The liquid film on the surface is broken within 10 seconds or more and less than 15 seconds.

D: The liquid film on the surface is broken within 1 second or more and less than 10 seconds.
E: The liquid film on the surface is instantaneously broken (within less than 1 second).

<Water content of substrate and medical device>

The substrate was immersed in a phosphate buffer solution and left to stand at room temperature for 24 hours or more. The substrate was pulled up from the phosphate buffer solution, the surface moisture was wiped off with a wiping cloth ("Kimwipe (registered trademark)"manufactured by NIPPON PAPER CRECIA CO., LTD.), and then the mass (Ww) of the substrate was measured. Then, the substrate was dried at 40°C for 2 hours with a vacuum dryer, and then the mass (Wd) was measured. From these masses, the water content of the substrate was calculated by the following formula (1). When the obtained value was less than 1%, it was judged as not more than the measurement limit, and was described as "less than 1%". The average of N = 3 was taken as the water content. The water content was similarly calculated for the substrate after sterilization, that is, the medical device.

$$\text{Water content (\%) of substrate} = 100 \times (Ww - Wd)/Ww$$

Formula (1)

<Amount of change of water content of substrate before and after sterilization>

From the measurement results of the water content of the substrate and the medical device, the amount of change of the water content was calculated by the following formula (2).

Amount of change (percentage point) of water content of substrate before and after sterilization = water content (mass%) of medical device - water content (mass%) of substrate    Formula (2)

<Friction coefficient>

Under the following conditions, the friction coefficient of the surface of the medical device in a state of being wetted with a phosphate buffer solution was measured at N = 5, and the average was taken as the friction coefficient.

Apparatus: Friction tester KES-SE (manufactured by KATO TECH CO., LTD.)
Friction SENS: H
Measurement SPEED: 2 × 1 mm/sec
Friction load: 44 g

<Lipid adhesion amount>

Into a 20 cc screw tube, 0.03 g of methyl palmitate, 10 g of pure water, and one contact lens-shaped sample were put. The screw tube was shaken at 37°C and 165 rpm for 3 hours. After shaking, the sample in the screw tube was scrubbed and washed with tap water at 40°C and a household liquid detergent ("Mama Lemon (registered trademark)" manufactured by Lion Corporation). The washed sample was placed in a screw tube containing a phosphate buffer solution, and stored in a

refrigerator at 4°C for 1 hour. Then, the sample was visually observed, and if there was a cloudy portion, it was determined that methyl palmitate adhered to the sample. The area of the portion to which methyl palmitate adhered with respect to the entire surface of the sample was observed.

<Tensile elastic modulus>

A test piece having a width (smallest portion) of 5 mm and a length of 14 mm was cut out from each of contact lens-shaped and sheet-shaped substrates using a prescribed blanking die. The test piece was subjected to a tensile test using TENSILON model RTG-1210 manufactured by A & D Company, Limited. The tensile speed was 100 mm/min, and the distance between grips (initial) was 5 mm. Measurements were made on both the substrate before sterilization and the medical device after sterilization. The measurement was performed at N = 8, and the average of the values of N = 6 excluding the maximum value and the minimum value was taken as the tensile elastic modulus.

<Tensile elastic modulus change rate of substrate before and after sterilization>

From the measurement results of the tensile elastic modulus of the substrate and the medical device, the tensile elastic modulus change rate was calculated by the following formula (3). The average of N = 6 was taken as the tensile elastic modulus change rate before and after sterilization.

Tensile elastic modulus change rate (%) of substrate before and after sterilization = (tensile elastic modulus of medical device after sterilization - tensile elastic modulus of substrate before sterilization)/tensile elastic modulus of substrate before sterilization $\times$ 100 Formula (3)

<Size>

For the contact lens-shaped and sheet-shaped substrates (N = 3), the diameters were measured, and the average was taken as the size. The size was similarly measured for the substrate after sterilization, that is, the medical device.

<Size change rate before and after sterilization>

From the measurement results of the size of the substrate and the medical device, the size change rate was calculated by the following formula (4). The average of N = 3 was taken as the size change rate before and after sterilization.

Size change rate (%) before and after sterilization = (size of device after sterilization - size of substrate before sterilization)/size of substrate before sterilization $\times$ 100 Formula (4)

<Measurement of molecular weight>

The molecular weight of the hydrophilic polymer was measured under the following conditions.

Apparatus: Prominence GPC system manufactured by Shimadzu Corporation
Pump: LC-20AD
Autosampler: SIL-20AHT
Column oven: CTO-20A
Detector: RID-10A
Column: GMPWXL (7.8 mm (internal diameter) $\times$ 30 cm, particle size: 13 $\mu$m) manufactured by Tosoh Corporation
Solvent: water/methanol = 1/1 (0.1 N lithium nitrate added)
Flow rate: 0.5 mL/min
Measurement time: 30 minutes
Sample concentration: 0.1 to 0.3 mass%
Sample injection volume: 100 $\mu$L
Standard sample: a polyethylene oxide standard sample (0.1 kD to 1258 kD) manufactured by Agilent

<pH Measurement method>

The pH of a solution was measured using a pH meter Eutech pH 2700 (manufactured by Eutech Instruments). In the tables, the pH before sterilization of the aqueous packaging solution containing a hydrophilic polymer was measured after all the hydrophilic polymers were added to the solution described in each of the examples and comparative examples, and the solution was stirred at room temperature (20 to 23°C) for 30 minutes using a rotor so as to be uniform. In the tables, the "pH after sterilization" was determined by performing the sterilization treatment once, cooling the solution to room temperature (20 to 23°C), and measuring the pH immediately after that.

<Determination of separation of hydrophilic polymer layer>

Whether or not the hydrophilic polymer layer was separated was determined by observing a cross section of the medical device using a transmission electron microscope.

Apparatus: transmission electron microscope

Condition: acceleration voltage of 100 kV

Sample preparation: A sample was prepared by ultrathin sectioning using $RuO_4$ staining. When it is difficult to distinguish between the substrate and the hydrophilic polymer layer, $OsO_4$ staining may be additionally performed. In the examples, when the substrate was a silicone hydrogel-based substrate or a silicone-based substrate, $RuO_4$ staining was performed.

An ultramicrotome was used to produce the ultrathin section.

<Elemental composition analysis of hydrophilic polymer layer>

Elemental composition analysis of the hydrophilic polymer layer was performed according to scanning transmission electron microscopy and electron energy loss spectroscopy by analyzing a cross section of a medical device frozen in a water-containing state using a cryotransfer holder.

Apparatus: field emission electron microscope
Acceleration voltage: 200 kV
Measurement temperature: about -100°C
Electron energy loss spectroscopy: GATAN GIF Tridiem
Image capturing: Digital Micrograph
Sample preparation: A sample was prepared by ultrathin sectioning using $RuO_4$ staining. When it is difficult to distinguish between the substrate and a coat layer, $OsO_4$ staining may be additionally performed. In the examples, when the substrate was a silicone hydrogel-based substrate or a silicone-based substrate, $RuO_4$ staining was performed.

An ultramicrotome was used to produce the ultrathin section.

<Film thickness of hydrophilic polymer layer>

A cross section of the medical device in a dry state was observed using a transmission electron microscope. Measurement was performed under the conditions described in <Determination of separation of hydrophilic polymer layer>. The film thickness was measured at one point per visual field in three different points, and the average of the film thicknesses at the total of three points was described.

[Production Example 1]

First, 28 parts by mass of polydimethylsiloxane having methacryloyl groups at both ends and represented by a formula (M1) (FM-7726, JNC CORPORATION, Mw: 30,000), 7 parts by mass of a silicone monomer represented by a formula (M2) (FM-0721, JNC Corporation, Mw: 5,000), 57.9 parts by mass of trifluoroethyl acrylate ("Viscoat (registered trademark)" 3F, OSAKA ORGANIC CHEMICAL INDUSTRY LTD.), 7 parts by mass of 2-ethylhexyl acrylate (Tokyo Chemical Industry Co., Ltd.), and 0.1 parts by mass of dimethylaminoethyl acrylate (Kohjin Co., Ltd.) were prepared. Then, 5,000 ppm of a photoinitiator "Irgacure (registered trademark)" 819 (NAGASE & CO., LTD.), 5,000 ppm of an ultraviolet absorber (RUVA-93, Otsuka Chemical Co., Ltd.), and 100 ppm of a colorant (RB246, Arran chemical) with respect to the total mass of the above-mentioned monomers were prepared, and 10 parts by mass of t-amyl alcohol with respect to 100 parts by mass in total of the above-mentioned monomers were further prepared, and all the components were mixed and stirred. The stirred mixture was filtered through a membrane filter (pore size: 0.45 μm) to remove insoluble matters, thereby obtaining a monomer mixture.

The monomer mixture was injected into a contact lens mold made of a transparent resin (material on the base curve side: polypropylene, material on the front curve side: polypropylene), and polymerized by light irradiation (wavelength: 405 nm (± 5 nm), illuminance: 0 to 0.7 mW/cm$^2$, 30 minutes) to produce a molded article containing a silicon atom-containing low water content soft material.

After the polymerization, the resulting molded article, together with the mold including the front curve mold and the base curve mold released from each other, was immersed in a 100 mass% aqueous solution of isopropyl alcohol at 60°C for 1.5 hours to peel off the contact lens-shaped molded article from the mold. The obtained molded article was immersed in a large excess amount of a 100 mass% aqueous solution of isopropyl alcohol kept at 60°C for 2 hours to extract impurities such as residual monomers. Then, the molded article was dried at room temperature (23°C) for 12 hours.

[Chemical Formula 1]

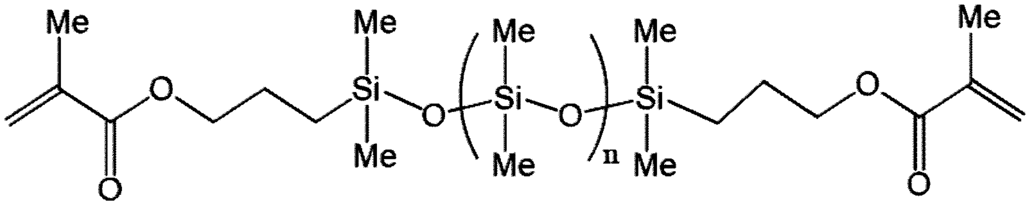
(M1)

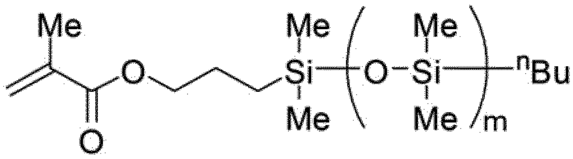
(M2)

<Phosphate buffer solution>

The phosphate buffer solution used in the processes of the following examples and comparative examples and the above-mentioned measurements has the following composition. In the following composition, EDTA2Na represents disodium dihydrogen ethylenediaminetetraacetate.

| | |
|---|---|
| KCl | 0.2 g/L |
| $KH_2PO_4$ | 0.2 g/L |
| NaCl | 8.0 g/L |
| $Na_2HPO_4$ | 1.19 g/L |
| EDTA2Na | 0.5 g/L |

[Example 1]

As a substrate, a commercially available silicone hydrogel lens "MyDay (registered trademark)" (manufactured by CooperVision Japan, Inc., stenfilcon A) containing silicone as a main component was used. The substrate was immersed in a solution obtained by adding 0.05 mass% of an acrylic acid/N,N-diethylacrylamide copolymer (molar ratio of copolymerization: 1/9, Mw: 280,000, manufactured by OSAKA ORGANIC CHEMICAL INDUSTRY LTD.) to a phosphate buffer solution, and sterilized in an autoclave at 121°C for 30 minutes. The results of evaluating the obtained medical device by the above-mentioned methods are shown in Tables 1 to 3.

[Example 2]

As a substrate, a commercially available silicone hydrogel lens "MyDay (registered trademark)" (manufactured by CooperVision Japan, Inc., stenfilcon A) containing silicone as a main component was used. The substrate was immersed in a solution obtained by adding 0.05 mass% of poly(N,N-diethylacrylamide) (Mw: 290,000, manufactured by OSAKA ORGANIC CHEMICAL INDUSTRY LTD.) to a phosphate buffer solution, and sterilized in an autoclave at 121°C for 30 minutes. The results of evaluating the obtained medical device by the above-mentioned methods are shown in Tables 1 to 3.

[Example 3]

As a substrate, a commercially available silicone hydrogel lens "MyDay (registered trademark)" (manufactured by CooperVision Japan, Inc., stenfilcon A) containing silicone as a main component was used. The substrate was immersed in a solution obtained by adding 0.04 mass% of an acrylic acid/N,N-diethylacrylamide copolymer (molar ratio of copolymerization: 1/9, Mw: 280,000, manufactured by OSAKA ORGANIC CHEMICAL INDUSTRY LTD.) to a phosphate buffer solution, and sterilized in an autoclave at 121°C for 30 minutes. The results of evaluating the obtained medical device by the above-mentioned methods are shown in Tables 1 to 3.

[Example 4]

As a substrate, a commercially available silicone hydrogel lens "MyDay (registered trademark)" (manufactured by CooperVision Japan, Inc., stenfilcon A) containing silicone as a main component was used. The substrate was immersed in a solution obtained by adding 0.04 mass% of poly(N,N-diethylacrylamide) (Mw: 290,000, manufactured by OSAKA ORGANIC CHEMICAL INDUSTRY LTD.) to a phosphate buffer solution, and sterilized in an autoclave at 121°C for 30 minutes. The results of evaluating the obtained medical device by the above-mentioned methods are shown in Tables 1 to 3.

[Example 5]

As a substrate, a commercially available silicone hydrogel lens "ACUVUE OASYS (registered trademark)" (manufactured by Johnson & Johnson K.K., senofilcon A) containing polyvinylpyrrolidone and silicone as main components was used. The substrate was immersed in a solution obtained by adding 0.05 mass% of an acrylic acid/N,N-diethylacrylamide copolymer (molar ratio of copolymerization: 1/9, Mw: 280,000, manufactured by OSAKA ORGANIC CHEMICAL INDUSTRY LTD.) to a phosphate buffer solution, and sterilized in an autoclave at 121°C for 30 minutes. The results of evaluating the obtained medical device by the above-mentioned methods are shown in Tables 1 to 3.

[Example 6]

As a substrate, a commercially available silicone hydrogel lens "ACUVUE OASYS (registered trademark)" (manufactured by Johnson & Johnson K.K., senofilcon A) containing polyvinylpyrrolidone and silicone as main components was used. The substrate was immersed in a solution obtained by adding 0.05 mass% of poly (N,N-diethylacrylamide) (Mw: 290,000, manufactured by OSAKA ORGANIC CHEMICAL INDUSTRY LTD.) to a phosphate buffer solution, and sterilized in an autoclave at 121°C for 30 minutes. The results of evaluating the obtained medical device by the above-mentioned methods are shown in Tables 1 to 3.

[Example 7]

As a substrate, a commercially available silicone hydrogel lens "ACUVUE OASYS (registered trademark)" (manufactured by Johnson & Johnson K.K., senofilcon A) containing polyvinylpyrrolidone and silicone as main components was used. The substrate was immersed in a solution obtained by adding 0.04 mass% of poly (N,N-diethylacrylamide) (Mw: 290,000, manufactured by OSAKA ORGANIC CHEMICAL INDUSTRY LTD.) to a phosphate buffer solution, and sterilized in an autoclave at 121°C for 30 minutes. The results of evaluating the obtained medical device by the above-mentioned methods are shown in Tables 1 to 3.

[Example 8]

As a substrate, the molded article obtained in Production Example 1 was used. The substrate was immersed in a solution obtained by adding 0.04 mass% of an acrylic acid/N,N-diethylacrylamide copolymer (molar ratio of copolymerization: 1/9, Mw: 280,000, manufactured by OSAKA ORGANIC CHEMICAL INDUSTRY LTD.) to a phosphate buffer solution, and sterilized in an autoclave at 121°C for 30 minutes. The results of evaluating the obtained medical device by the above-mentioned methods are shown in Tables 1 to 3.

[Example 9]

As a substrate, the molded article obtained in Production Example 1 was used. The substrate was immersed in a solution obtained by adding 0.04 mass% of poly(N,N-diethylacrylamide) (Mw: 290,000, manufactured by OSAKA ORGANIC CHEMICAL INDUSTRY LTD.) to a phosphate buffer solution, and sterilized in an autoclave at 121°C for 30 minutes. The results of evaluating the obtained medical device by the above-mentioned methods are shown in Tables 1 to 3.

[Example 10]

As a substrate, a commercially available PF catheter tube (manufactured and sold by Toray Industries, Inc.) containing polyurethane as a main component was used. The substrate was immersed in a solution obtained by adding 0.04 mass% of an acrylic acid/N,N-diethylacrylamide copolymer (molar ratio of copolymerization: 1/9, Mw: 800,000, manufactured by OSAKA ORGANIC CHEMICAL INDUSTRY LTD.) to a phosphate buffer solution, and heated in an

autoclave at 121°C for 30 minutes. The results of evaluating the obtained medical device by the above-mentioned methods are shown in Tables 1 to 3.

[Example 11]

As a substrate, a commercially available PF catheter tube (manufactured and sold by Toray Industries, Inc.) containing polyurethane as a main component was used. The substrate was immersed in a solution obtained by adding 0.04 mass% of poly(N,N-diethylacrylamide) (Mw: 290,000, manufactured by OSAKA ORGANIC CHEMICAL INDUSTRY LTD.) to a phosphate buffer solution, and heated in an autoclave at 121°C for 30 minutes. The results of evaluating the obtained medical device by the above-mentioned methods are shown in Tables 1 to 3.

[Example 12]

As a substrate, a commercially available film "MICTRON (registered trademark)" (manufactured by Toray Industries, Inc.) containing a para-type aromatic polyamide (aramid) as a main component was used. The substrate was immersed in a solution obtained by adding 0.04 mass% of an acrylic acid/N,N-diethylacrylamide copolymer (molar ratio of copolymerization: 1/9, Mw: 800,000, manufactured by OSAKA ORGANIC CHEMICAL INDUSTRY LTD.) to a phosphate buffer solution, and heated in an autoclave at 121°C for 30 minutes. The results of evaluating the obtained medical device by the above-mentioned methods are shown in Tables 1 to 3.

[Example 13]

As a substrate, a commercially available film "MICTRON (registered trademark)" (manufactured by Toray Industries, Inc.) containing a para-type aromatic polyamide (aramid) as a main component was used. The substrate was immersed in a solution obtained by adding 0.04 mass% of poly(N,N-diethylacrylamide) (Mw: 290,000, manufactured by OSAKA ORGANIC CHEMICAL INDUSTRY LTD.) to a phosphate buffer solution, and heated in an autoclave at 121°C for 30 minutes. The results of evaluating the obtained medical device by the above-mentioned methods are shown in Tables 1 to 3.

[Example 14]

As a substrate, a commercially available hydrogel lens "1-DAY ACUVUE (registered trademark)" (manufactured by Johnson & Johnson K.K., etafilcon A) containing 2-hydroxyethyl methacrylate as a main component was used. The substrate was immersed in a solution obtained by adding 0.04 mass% of an acrylic acid/N,N-diethylacrylamide copolymer (molar ratio of copolymerization: 1/9, Mw: 280,000, manufactured by OSAKA ORGANIC CHEMICAL INDUSTRY LTD.) to a phosphate buffer solution, and sterilized in an autoclave at 121°C for 30 minutes. The results of evaluating the obtained medical device by the above-mentioned methods are shown in Tables 1 to 3.

[Example 15]

As a substrate, a commercially available hydrogel lens "1-DAY ACUVUE (registered trademark)" (manufactured by Johnson & Johnson K.K., etafilcon A) containing 2-hydroxyethyl methacrylate as a main component was used. The substrate was immersed in a solution obtained by adding 0.04 mass% of poly(N,N-diethylacrylamide) (Mw: 290,000, manufactured by OSAKA ORGANIC CHEMICAL INDUSTRY LTD.) to a phosphate buffer solution, and sterilized in an autoclave at 121°C for 30 minutes. The results of evaluating the obtained medical device by the above-mentioned methods are shown in Tables 1 to 3.

[Example 16]

As a substrate, a commercially available silicone hydrogel lens "ACUVUE OASYS (registered trademark)" (manufactured by Johnson & Johnson K.K., senofilcon A) containing polyvinylpyrrolidone and silicone as main components was used. The substrate was immersed in a solution obtained by adding 0.05 mass% of an N,N-dimethylacrylamide/N,N-diethylacrylamide copolymer (molar ratio of copolymerization: 1/9, Mw: 280,000, manufactured by OSAKA ORGANIC CHEMICAL INDUSTRY LTD.) to a phosphate buffer solution, and sterilized in an autoclave at 121°C for 30 minutes. The results of evaluating the obtained medical device by the above-mentioned methods are shown in Tables 1 to 3.

[Example 17]

As a substrate, a commercially available silicone hydrogel lens "ACUVUE OASYS (registered trademark)" (manufactured by Johnson & Johnson K.K., senofilcon A) containing polyvinylpyrrolidone and silicone as main components was used. The substrate was immersed in a solution obtained by adding 0.05 mass% of an acrylic acid/N,N-dimethylaminopropyl acrylamide methyl chloride quaternary salt/N,N-diethylacrylamide copolymer (molar ratio of copolymerization: 1/1/8, Mw: 280,000, manufactured by OSAKA ORGANIC CHEMICAL INDUSTRY LTD.) to a phosphate buffer solution, and sterilized in an autoclave at 121°C for 30 minutes. The results of evaluating the obtained medical device by the above-mentioned methods are shown in Tables 1 to 3.

[Example 18]

As a substrate, the molded article obtained in Production Example 1 was used. The substrate was immersed in a solution obtained by adding 0.05 mass% of an acrylic acid/N,N-diethylacrylamide copolymer (molar ratio of copolymerization: 1/9, Mw: 280,000, manufactured by OSAKA ORGANIC CHEMICAL INDUSTRY LTD.) to a phosphate buffer solution, and sterilized in an autoclave at 121°C for 30 minutes. The results of evaluating the obtained medical device by the above-mentioned methods are shown in Tables 1 to 3.

[Example 19]

As a substrate, the molded article obtained in Production Example 1 was used. The substrate was immersed in a solution obtained by adding 0.03 mass% of an acrylic acid/N,N-diethylacrylamide copolymer (molar ratio of copolymerization: 1/9, Mw: 280,000, manufactured by OSAKA ORGANIC CHEMICAL INDUSTRY LTD.) to a phosphate buffer solution, and sterilized in an autoclave at 121°C for 30 minutes. The results of evaluating the obtained medical device by the above-mentioned methods are shown in Tables 1 to 3.

[Table 1-1]

| | Substrate | Water content of substrate (mass%) | Hydrophilic polymer and concentration of hydrophilic polymer solution | pH Before sterilization | pH After sterilization |
|---|---|---|---|---|---|
| Example 1 | "MyDay (registered trademark)" | 54.0 | 0.05 mass% acrylic acid/N,N-diethylacrylamide copolymer | 7.0 | 7.1 |
| Example 2 | "MyDay (registered trademark)" | 54.0 | 0.05 mass% poly(N,N-diethylacrylamide) | 7.1 | 7.2 |
| Example 3 | "MyDay (registered trademark)" | 54.0 | 0.04 mass% acrylic acid/N,N-diethylacrylamide copolymer | 7.0 | 7.1 |
| Example 4 | "MyDay (registered trademark)" | 54.0 | 0.04 mass% poly(N,N-diethylacrylamide) | 7.1 | 7.2 |
| Example 5 | "ACUVUE OASYS (registered trademark)" | 38.0 | 0.05 mass% acrylic acid/N,N-diethylacrylamide copolymer | 7.0 | 7.1 |
| Example 6 | "ACUVUE OASYS (registered trademark)" | 38.0 | 0.05 mass% poly(N,N-diethylacrylamide) | 7.1 | 7.2 |
| Example 7 | "ACUVUE OASYS (registered trademark)" | 38.0 | 0.04 mass% poly(N,N-diethylacrylamide) | 7.1 | 7.2 |

[Table 1-2]

| | Substrate | Water content of substrate (mass%) | Hydrophilic polymer and concentration of hydrophilic polymer solution | pH Before sterilization | pH After sterilization |
|---|---|---|---|---|---|
| Example 8 | Production Example 1 | < 1 | 0.04 mass% acrylic acid/N,N-diethylacrylamide copolymer | 7.0 | 7.1 |
| Example 9 | Production Example 1 | < 1 | 0.04 mass% poly(N,N-diethylacrylamide) | 7.1 | 7.2 |
| Example 10 | PF catheter tube | < 1 | 0.04 mass% acrylic acid/N,N-diethylacrylamide copolymer | 7.0 | 7.1 |
| Example 11 | PF catheter tube | < 1 | 0.04 mass% poly(N,N-diethylacrylamide) | 7.1 | 7.2 |
| Example 12 | "MICTRON (registered trademark)" | < 1 | 0.04 mass% acrylic acid/N,N-diethylacrylamide copolymer | 7.0 | 7.2 |
| Example 13 | "MICTRON (registered trademark)" | < 1 | 0.04 mass% poly(N,N-diethylacrylamide) | 7.1 | 7.3 |

[Table 1-3]

| | Substrate | Water content of substrate (mass%) | Hydrophilic polymer and concentration of hydrophilic polymer solution | pH Before sterilization | pH After sterilization |
|---|---|---|---|---|---|
| Example 14 | "1-DAY ACUVUE (registered trademark)" | 58.0 | 0.04 mass% acrylic acid/N,N-diethylacrylamide copolymer | 7.0 | 7.1 |
| Example 15 | "1-DAY ACUVUE (registered trademark)" | 58.0 | 0.04 mass% poly(N,N-diethylacrylamide) | 7.1 | 7.2 |
| Example 16 | "ACUVUE OASYS (registered trademark)" | 38.0 | 0.05 mass% N, N-dimethylacrylamide/N,N-diethylacrylamide copolymer | 7.0 | 7.1 |
| Example 17 | "ACUVUE OASYS (registered trademark)" | 38.0 | 0.05 mass% acrylic acid/N,N-dimethylaminopropyl acrylamide methyl chloride quaternary salt/N,N-diethylacrylamide copolymer | 7.0 | 7.1 |
| Example 18 | Production Example 1 | < 1 | 0.05 mass% acrylic acid/N,N-diethylacrylamide copolymer | 7.0 | 7.1 |
| Example 19 | Production Example 1 | < 1 | 0.03 mass% acrylic acid/N,N-diethylacrylamide copolymer | 7.0 | 7.0 |

[Table 2-1]

| | Liquid film retention time (sec) | Liquid film retention time 2 hours after scrubbing and washing (sec) | Water content of medical device (mass%) | Results of elemental composition analysis of hydrophilic polymer layer | Film thickness of hydrophilic polymer layer (nm) | Amount of change of water content (percentage point) | Friction coefficient |
|---|---|---|---|---|---|---|---|
| Example 1 | A (120 sec) | A (120 sec) | 54.5 | Coating polymer was separated into two layers (one of layers is mixed with substrate) | 15 | 0.5 | 0.205 |
| Example 2 | A (120 sec) | A (120 sec) | 54.3 | Coating polymer was separated into two layers (one of layers is mixed with substrate) | 16 | 0.3 | 0.249 |
| Example 3 | A (120 sec) | A (120 sec) | 54.2 | Coating polymer was separated into two layers (one of layers is mixed with substrate) | 13 | 0.2 | 0.224 |
| Example 4 | A (120 sec) | A (120 sec) | 54.2 | Coating polymer was separated into two layers (one of layers is mixed with substrate) | 10 | 0.2 | 0.289 |
| Example 5 | A (120 sec) | A (120 sec) | 38.2 | Coating polymer was separated into two layers (one of layers is mixed with substrate) | 12 | 0.2 | 0.150 |
| Example 6 | A (120 sec) | A (120 sec) | 38.5 | Coating polymer was separated into two layers (one of layers is mixed with substrate) | 10 | 0.5 | 0.179 |
| Example 7 | A (120 sec) | A (120 sec) | 38.3 | Coating polymer mixed with substrate, and coating polymer alone | 9 | 0.3 | 0.250 |

[Table 2-2]

| | Liquid film retention time (sec) | Liquid film retention time 2 hours after scrubbing and washing (sec) | Water content of medical device (mass%) | Results of elemental composition analysis of hydrophilic polymer layer | Film thickness of hydrophilic polymer layer (nm) | Amount of change of water content (percentage point) | Friction coefficient |
|---|---|---|---|---|---|---|---|
| Example 8 | A (60 sec) | A (60 sec) | 1.9 | Coating polymer mixed with substrate, and coating polymer alone | 10 | 1.9 | 0.245 |
| Example 9 | A (60 sec) | A (60 sec) | 1.5 | Coating polymer mixed with substrate, and coating polymer alone | 11 | 1.5 | 0.179 |
| Example 10 | A (120 sec) | A (120 sec) | 0.9 | Coating polymer mixed with substrate, and coating polymer alone | 9 | 0.9 | Not measurable because medical device had tubular shape |
| Example 11 | A (120 sec) | A (120 sec) | 0.9 | Coating polymer mixed with substrate, and coating polymer alone | 8 | 0.9 | Not measurable because medical device had tubular shape |
| Example 12 | B (15 sec) | B (15 sec) | 0.3 | Coating polymer mixed with substrate, and coating polymer alone | 5 | 0.3 | Not measurable because medical device had sheet shape |
| Example 13 | B (15 sec) | B (15 sec) | 0.4 | Coating polymer mixed with substrate, and coating polymer alone | 4 | 0.4 | Not measurable because medical device had sheet shape |

[Table 2-3]

| | Liquid film retention time (sec) | Liquid film retention time 2 hours after scrubbing and washing (sec) | Water content of medical device (mass%) | Results of elemental composition analysis of hydrophilic polymer layer | Film thickness of hydrophilic polymer layer (nm) | Amount of change of water content (percentage point) | Friction coefficient |
|---|---|---|---|---|---|---|---|
| Example 14 | A (120 sec) | A (120 sec) | 58.3 | Coating polymer mixed with substrate, and coating polymer alone | 10 | 0.3 | 0.290 |
| Example 15 | A (120 sec) | A (120 sec) | 58.4 | Coating polymer mixed with substrate, and coating polymer alone | 10 | 0.4 | 0.283 |
| Example 16 | A (120 sec) | A (120 sec) | 38.6 | Coating polymer mixed with substrate, and coating polymer alone | 15 | 0.6 | 0.149 |
| Example 17 | A (120 sec) | A (120 sec) | 38.5 | Coating polymer mixed with substrate, and coating polymer alone | 12 | 0.5 | 0.138 |
| Example 18 | A (120 sec) | A (120 sec) | 1.8 | Coating polymer mixed with substrate, and coating polymer alone | 15 | 1.8 | 0.175 |
| Example 19 | A (60 sec) | A (60 sec) | 1.5 | Coating polymer mixed with substrate, and coating polymer alone | 12 | 1.5 | 0.170 |

[Table 3-1]

| | Lipid adhesion amount | Tensile elastic modulus of substrate (MPa) | Tensile elastic modulus of medical device (MPa) | Tensile elastic modulus change rate (%) | Size of substrate (mm) | Size of medical device (mm) | Size change rate (%) |
|---|---|---|---|---|---|---|---|
| Example 1 | No adhesion | 0.61 | 0.61 | 0.2 | 14.20 | 14.21 | 0.07 |
| Example 2 | No adhesion | 0.61 | 0.60 | -0.9 | 14.20 | 14.22 | 0.14 |

(continued)

| | Lipid adhesion amount | Tensile elastic modulus of substrate (MPa) | Tensile elastic modulus of medical device (MPa) | Tensile elastic modulus change rate (%) | Size of substrate (mm) | Size of medical device (mm) | Size change rate (%) |
|---|---|---|---|---|---|---|---|
| Example 3 | No adhesion | 0.61 | 0.61 | 0.7 | 14.20 | 14.16 | -0.28 |
| Example 4 | No adhesion | 0.61 | 0.62 | 1.4 | 14.20 | 14.15 | -0.35 |
| Example 5 | No adhesion | 0.70 | 0.72 | 2.9 | 14.20 | 14.21 | 0.07 |
| Example 6 | No adhesion | 0.70 | 0.73 | 4.3 | 14.20 | 14.31 | 0.77 |
| Example 7 | No adhesion | 0.70 | 0.72 | 2.9 | 14.20 | 14.22 | 0.14 |

[Table 3-2]

| | Lipid adhesion amount | Tensile elastic modulus of substrate (MPa) | Tensile elastic modulus of medical device (MPa) | Tensile elastic modulus change rate (%) | Size of substrate (mm) | Size of medical device (mm) | Size change rate (%) |
|---|---|---|---|---|---|---|---|
| Example 8 | No adhesion | 0.53 | 0.55 | 3.7 | 14.20 | 14.15 | -0.35 |
| Example 9 | No adhesion | 0.53 | 0.56 | 5.6 | 14.20 | 14.18 | -0.14 |
| Example 10 | No adhesion | Not measurable because medical device had tubular shape | Not measurable because medical device had tubular shape | Not measurable because medical device had tubular shape | Not measurable because medical device had tubular shape | Not measurable because medical device had tubular shape | Not measurable because medical device had tubular shape |
| Example 11 | No adhesion | Not measurable because medical device had tubular shape | Not measurable because medical device had tubular shape | Not measurable because medical device had tubular shape | Not measurable because medical device had tubular shape | Not measurable because medical device had tubular shape | Not measurable because medical device had tubular shape |
| Example 12 | No adhesion | 1. 10 | 1.15 | 4.5 | 15.00 | 15.10 | 0.66 |
| Example 13 | No adhesion | 1. 10 | 1.15 | 4.5 | 15.00 | 15.10 | 0.66 |

[Table 3-3]

| | Lipid adhesion amount | Tensile elastic modulus of substrate (MPa) | Tensile elastic modulus of medical device (MPa) | Tensile elastic modulus change rate (%) | Size of substrate (mm) | Size of medical device (mm) | Size change rate (%) |
|---|---|---|---|---|---|---|---|
| Example 14 | No adhesion | 0.3 | 0.31 | 3.3 | 14.20 | 14.22 | 0.14 |
| Example 15 | No adhesion | 0.3 | 0.31 | 3.3 | 14.20 | 14.28 | 0.56 |

(continued)

| | Lipid adhesion amount | Tensile elastic modulus of substrate (MPa) | Tensile elastic modulus of medical device (MPa) | Tensile elastic modulus change rate (%) | Size of substrate (mm) | Size of medical device (mm) | Size change rate (%) |
|---|---|---|---|---|---|---|---|
| Example 16 | No adhesion | 0.70 | 0.72 | 2. 9 | 14.20 | 14.32 | 0.84 |
| Example 17 | No adhesion | 0.70 | 0.72 | 2.9 | 14.20 | 14.29 | 0.63 |
| Example 18 | No adhesion | 0.53 | 0.55 | 3.7 | 14.20 | 14.19 | -0.07 |
| Example 19 | No adhesion | 0.53 | 0.55 | 3.7 | 14.20 | 14.21 | 0.07 |

[Comparative Example 1]

A commercially available silicone hydrogel lens "MyDay (registered trademark)" (manufactured by CooperVision Japan, Inc., stenfilcon A) containing silicone as a main component was immersed in a phosphate buffer solution, and sterilized in an autoclave at 121°C for 30 minutes. The results of evaluating the obtained medical device (no hydrophilic polymer layer was observed) by the above-mentioned methods are shown in Tables 4 to 6.

[Comparative Example 2]

As a substrate, a commercially available silicone hydrogel lens "MyDay (registered trademark)" (manufactured by CooperVision Japan, Inc., stenfilcon A) containing silicone as a main component was used. The substrate was immersed in a solution obtained by adding 0.03 mass% of an acrylic acid/acrylamide copolymer (molar ratio of copolymerization: 1/9, Mw: 250,000, internally manufactured) to a phosphate buffer solution, and sterilized in an autoclave at 121°C for 30 minutes. The results of evaluating the obtained medical device (no hydrophilic polymer layer was observed) by the above-mentioned methods are shown in Tables 4 to 6.

[Comparative Example 3]

As a substrate, a commercially available silicone hydrogel lens "MyDay (registered trademark)" (manufactured by CooperVision Japan, Inc., stenfilcon A) containing silicone as a main component was used. The substrate was immersed in a solution obtained by adding 0.03 mass% of an acrylic acid/N,N-dimethylacrylamide copolymer (molar ratio of copolymerization: 1/9, Mw: 200,000, manufactured by OSAKA ORGANIC CHEMICAL INDUSTRY LTD.) to a phosphate buffer solution, and sterilized in an autoclave at 121°C for 30 minutes. The results of evaluating the obtained medical device (no hydrophilic polymer layer was observed) by the above-mentioned methods are shown in Tables 4 to 6.

[Comparative Example 4]

As a substrate, a commercially available silicone hydrogel lens "MyDay (registered trademark)" (manufactured by CooperVision Japan, Inc., stenfilcon A) containing silicone as a main component was used. The substrate was immersed in a solution obtained by adding 0.03 mass% of an acrylic acid/N,N-dimethylacrylamide copolymer (molar ratio of copolymerization: 1/9, Mw: 800,000, manufactured by OSAKA ORGANIC CHEMICAL INDUSTRY LTD.) to a phosphate buffer solution, and sterilized in an autoclave at 121°C for 30 minutes. The results of evaluating the obtained medical device (no hydrophilic polymer layer was observed) by the above-mentioned methods are shown in Tables 4 to 6.

[Comparative Example 5]

As a substrate, a commercially available silicone hydrogel lens "MyDay (registered trademark)" (manufactured by CooperVision Japan, Inc., stenfilcon A) containing silicone as a main component was used. The substrate was immersed in a solution obtained by adding 0.03 mass% of an acrylic acid/acryloylmorpholine copolymer (molar ratio of copolymerization: 1/9, Mw: 500,000, manufactured by OSAKA ORGANIC CHEMICAL INDUSTRY LTD.) to a phosphate buffer solution, and sterilized in an autoclave at 121°C for 30 minutes. The results of evaluating the obtained medical device (no hydrophilic polymer layer was observed) by the above-mentioned methods are shown in Tables 4 to 6.

[Comparative Example 6]

As a substrate, a commercially available silicone hydrogel lens "MyDay (registered trademark)" (manufactured by CooperVision Japan, Inc., stenfilcon A) containing silicone as a main component was used. The substrate was immersed in a solution obtained by adding 0.03 mass% of an acrylic acid/acryloylmorpholine copolymer (molar ratio of copolymerization: 1/9, Mw: 320,000, manufactured by OSAKA ORGANIC CHEMICAL INDUSTRY LTD.) to a phosphate buffer solution, and sterilized in an autoclave at 121°C for 30 minutes. The results of evaluating the obtained medical device (no hydrophilic polymer layer was observed) by the above-mentioned methods are shown in Tables 4 to 6.

[Comparative Example 7]

As a substrate, a commercially available silicone hydrogel lens "ACUVUE OASYS (registered trademark)" (manufactured by Johnson & Johnson K.K., senofilcon A) containing polyvinylpyrrolidone and silicone as main components was used. The substrate was immersed in a solution obtained by adding 0.03 mass% of an acrylic acid/vinylpyrrolidone/N,N-dimethylacrylamide copolymer (molar ratio of copolymerization: 1/1/2, Mw: 550,000, manufactured by OSAKA ORGANIC CHEMICAL INDUSTRY LTD.) to a phosphate buffer solution, and sterilized in an autoclave at 121°C for 30 minutes. The results of evaluating the obtained medical device (no hydrophilic polymer layer was observed) by the above-mentioned methods are shown in Tables 4 to 6.

[Comparative Example 8]

As a substrate, a commercially available silicone hydrogel lens "ACUVUE OASYS (registered trademark)" (manufactured by Johnson & Johnson K.K., senofilcon A) containing polyvinylpyrrolidone and silicone as main components was used. The substrate was immersed in a solution obtained by adding 0.03 mass% of an acrylic acid/vinylpyrrolidone/N,N-dimethylacrylamide copolymer (molar ratio of copolymerization: 1/1/2, Mw: 330,000, manufactured by OSAKA ORGANIC CHEMICAL INDUSTRY LTD.) to a phosphate buffer solution, and sterilized in an autoclave at 121°C for 30 minutes. The results of evaluating the obtained medical device (no hydrophilic polymer layer was observed) by the above-mentioned methods are shown in Tables 4 to 6.

[Comparative Example 9]

As a substrate, a commercially available silicone hydrogel lens "ACUVUE OASYS (registered trademark)" (manufactured by Johnson & Johnson K.K., senofilcon A) containing polyvinylpyrrolidone and silicone as main components was used. The substrate was immersed in a solution obtained by adding 0.03 mass% of an acrylic acid/vinylpyrrolidone/N,N-dimethylacrylamide copolymer (molar ratio of copolymerization: 1/1/8, Mw: 500,000, manufactured by OSAKA ORGANIC CHEMICAL INDUSTRY LTD.) to a phosphate buffer solution, and sterilized in an autoclave at 121°C for 30 minutes. The results of evaluating the obtained medical device (no hydrophilic polymer layer was observed) by the above-mentioned methods are shown in Tables 4 to 6.

[Comparative Example 10]

As a substrate, a commercially available silicone hydrogel lens "ACUVUE OASYS (registered trademark)" (manufactured by Johnson & Johnson K.K., senofilcon A) containing polyvinylpyrrolidone and silicone as main components was used. The substrate was immersed in a solution obtained by adding 0.03 mass% of an acrylic acid/vinylpyrrolidone/N,N-dimethylacrylamide copolymer (molar ratio of copolymerization: 1/1/8, Mw: 370,000, manufactured by OSAKA ORGANIC CHEMICAL INDUSTRY LTD.) to a phosphate buffer solution, and sterilized in an autoclave at 121°C for 30 minutes. The results of evaluating the obtained medical device (no hydrophilic polymer layer was observed) by the above-mentioned methods are shown in Tables 4 to 6.

[Comparative Example 11]

As a substrate, a commercially available silicone hydrogel lens "ACUVUE OASYS (registered trademark)" (manufactured by Johnson & Johnson K.K., senofilcon A) containing polyvinylpyrrolidone and silicone as main components was used. The substrate was immersed in a solution obtained by adding 0.03 mass% of an acrylic acid/vinylpyrrolidone copolymer (molar ratio of copolymerization: 1/4, Mw: 590,000, manufactured by OSAKA ORGANIC CHEMICAL INDUSTRY LTD.) to a phosphate buffer solution, and sterilized in an autoclave at 121°C for 30 minutes. The results of evaluating the obtained medical device (no hydrophilic polymer layer was observed) by the above-mentioned methods are shown in Tables 4 to 6.

[Comparative Example 12]

As a substrate, a commercially available silicone hydrogel lens "ACUVUE OASYS (registered trademark)" (manufactured by Johnson & Johnson K.K., senofilcon A) containing polyvinylpyrrolidone and silicone as main components was used. The substrate was immersed in a solution obtained by adding 0.03 mass% of an acrylic acid/vinylpyrrolidone copolymer (molar ratio of copolymerization: 1/9, Mw: 390,000, manufactured by OSAKA ORGANIC CHEMICAL INDUSTRY LTD.) to a phosphate buffer solution, and sterilized in an autoclave at 121°C for 30 minutes. The results of evaluating the obtained medical device (no hydrophilic polymer layer was observed) by the above-mentioned methods are shown in Tables 4 to 6.

[Comparative Example 13]

As a substrate, a commercially available silicone hydrogel lens "ACUVUE OASYS (registered trademark)" (manufactured by Johnson & Johnson K.K., senofilcon A) containing polyvinylpyrrolidone and silicone as main components was used. The substrate was immersed in a solution obtained by adding 0.03 mass% of an acrylic acid/2-hydroxyethyl methacrylate/N,N-dimethylacrylamide copolymer (molar ratio of copolymerization: 1/1/2, Mw: 430,000, manufactured by OSAKA ORGANIC CHEMICAL INDUSTRY LTD.) to a phosphate buffer solution, and sterilized in an autoclave at 121°C for 30 minutes. The results of evaluating the obtained medical device (no hydrophilic polymer layer was observed) by the above-mentioned methods are shown in Tables 4 to 6.

[Comparative Example 14]

As a substrate, a commercially available silicone hydrogel lens "ACUVUE OASYS (registered trademark)" (manufactured by Johnson & Johnson K.K., senofilcon A) containing polyvinylpyrrolidone and silicone as main components was used. The substrate was immersed in a solution obtained by adding 0.03 mass% of an acrylic acid/2-hydroxyethyl methacrylate/N,N-dimethylacrylamide copolymer (molar ratio of copolymerization: 1/1/8, Mw: 480,000, manufactured by OSAKA ORGANIC CHEMICAL INDUSTRY LTD.) to a phosphate buffer solution, and sterilized in an autoclave at 121°C for 30 minutes. The results of evaluating the obtained medical device (no hydrophilic polymer layer was observed) by the above-mentioned methods are shown in Tables 4 to 6.

[Comparative Example 15]

As a substrate, a commercially available silicone hydrogel lens "ACUVUE OASYS (registered trademark)" (manufactured by Johnson & Johnson K.K., senofilcon A) containing polyvinylpyrrolidone and silicone as main components was used. The substrate was immersed in a solution obtained by adding 0.03 mass% of polyvinylpyrrolidone (Mw: 360, 000, manufactured by OSAKA ORGANIC CHEMICAL INDUSTRY LTD.) to a phosphate buffer solution, and sterilized in an autoclave at 121°C for 30 minutes. The results of evaluating the obtained medical device (no hydrophilic polymer layer was observed) by the above-mentioned methods are shown in Tables 4 to 6.

[Comparative Example 16]

As a substrate, a commercially available silicone hydrogel lens "ACUVUE OASYS (registered trademark)" (manufactured by Johnson & Johnson K.K., senofilcon A) containing polyvinylpyrrolidone and silicone as main components was used. The substrate was immersed in a solution obtained by adding 0.03 mass% of poly(N,N-dimethylacrylamide) (Mw: 360,000, manufactured by OSAKA ORGANIC CHEMICAL INDUSTRY LTD.) to a phosphate buffer solution, and sterilized in an autoclave at 121°C for 30 minutes. The results of evaluating the obtained medical device (no hydrophilic polymer layer was observed) by the above-mentioned methods are shown in Tables 4 to 6.

[Comparative Example 17]

As a substrate, a commercially available silicone hydrogel lens "ACUVUE OASYS (registered trademark)" (manufactured by Johnson & Johnson K.K., senofilcon A) containing polyvinylpyrrolidone and silicone as main components was immersed in a phosphate buffer solution, and sterilized in an autoclave at 121°C for 30 minutes. The results of evaluating the obtained medical device (no hydrophilic polymer layer was observed) by the above-mentioned methods are shown in Tables 4 to 6.

[Comparative Example 18]

As a substrate, the molded article obtained in Production Example 1 was immersed in a phosphate buffer solution, and sterilized in an autoclave at 121°C for 30 minutes. The results of evaluating the obtained medical device (no hydrophilic polymer layer was observed) by the above-mentioned methods are shown in Tables 4 to 6.

[Comparative Example 19]

As a substrate, a commercially available hydrogel lens "1-DAY ACUVUE (registered trademark)" (manufactured by Johnson & Johnson K.K., etafilcon A) containing 2-hydroxyethyl methacrylate as a main component was immersed in a phosphate buffer solution, and sterilized in an autoclave at 121°C for 30 minutes. The results of evaluating the obtained medical device (no hydrophilic polymer layer was observed) by the above-mentioned methods are shown in Tables 4 to 6.
The substrate of this comparative example had a relatively satisfactory liquid film retention time and had good durability even without the application of the manufacturing method of the present invention. However, in comparison with Examples 14 and 15 in which the manufacturing method of the present invention was applied to the same substrate, it was shown that application of the manufacturing method of the present invention significantly improves the liquid film retention time (20 seconds → 120 seconds).

[Comparative Example 20]

As a substrate, a commercially available PF catheter tube (manufactured and sold by Toray Industries, Inc.) containing polyurethane as a main component was immersed in a phosphate buffer solution, and sterilized in an autoclave at 121°C for 30 minutes. The results of evaluating the obtained medical device (no hydrophilic polymer layer was observed) by the above-mentioned methods are shown in Tables 4 to 6.

[Comparative Example 21]

As a substrate, a commercially available film "MICTRON (registered trademark)" (manufactured by Toray Industries, Inc.) containing a para-type aromatic polyamide (aramid) as a main component was immersed in a phosphate buffer solution, and sterilized in an autoclave at 121°C for 30 minutes. The results of evaluating the obtained medical device (no hydrophilic polymer layer was observed) by the above-mentioned methods are shown in Tables 4 to 6.

[Table 4-1]

| | Substrate | Water content of substrate (mass%) | Hydrophilic polymer and concentration of hydrophilic polymer solution | pH Before sterilization | pH After sterilization |
|---|---|---|---|---|---|
| Comparative Example 1 | "MyDay (registered trademark)" | 54.0 | No | 7.0 | 7.2 |
| Comparative Example 2 | "MyDay (registered trademark)" | 54.0 | 0.03 mass% acrylic acid/acrylamide copolymer | 7.0 | 7.2 |
| Comparative Example 3 | "MyDay (registered trademark)" | 54.0 | 0.03 mass% acrylic acid/N,N-dimethylacrylamide copolymer | 7.0 | 7.1 |
| Comparative Example 4 | "MyDay (registered trademark)" | 54.0 | 0.03 mass% acrylic acid/N,N-dimethylacrylamide copolymer | 7.0 | 7.1 |
| Comparative Example 5 | "MyDay (registered trademark)" | 54.0 | 0.03 mass% acrylic acid/acryloylmorpholine copolymer | 7.0 | 7.2 |
| Comparative Example 6 | "MyDay (registered trademark)" | 54.0 | 0.03 mass% acrylic acid/acryloylmorpholine copolymer | 7.0 | 7.2 |

(continued)

| | Substrate | Water content of substrate (mass%) | Hydrophilic polymer and concentration of hydrophilic polymer solution | pH Before sterilization | pH After sterilization |
|---|---|---|---|---|---|
| Comparative Example 7 | "ACUVUE OASYS (registered trademark)" | 38.0 | 0.03 mass% acrylic acid/vinylpyrrolidone/N,N-dimethylacrylamide copolymer | 7.0 | 7.0 |

[Table 4-2]

| | Substrate | Water content of substrate (mass%) | Hydrophilic polymer and concentration of hydrophilic polymer solution | pH Before sterilization | pH After sterilization |
|---|---|---|---|---|---|
| Comparative Example 8 | "ACUVUE OASYS (registered trademark)" | 38.0 | 0.03 mass% acrylic acid/vinylpyrrolidone/N,N-dimethylacrylamide copolymer | 7.0 | 7.1 |
| Comparative Example 9 | "ACUVUE OASYS (registered trademark)" | 38.0 | 0.03 mass% acrylic acid/vinylpyrrolidone/N,N-dimethylacrylamide copolymer | 7.0 | 7.2 |
| Comparative Example 10 | "ACUVUE OASYS (registered trademark)" | 38.0 | 0.03 mass% acrylic acid/vinylpyrrolidone/N,N-dimethylacrylamide copolymer | 7.0 | 7.2 |
| Comparative Example 11 | "ACUVUE OASYS (registered trademark)" | 38.0 | 0.03 mass% acrylic acid/vinylpyrrolidone copolymer | 7.0 | 7.1 |
| Comparative Example 12 | "ACUVUE OASYS (registered trademark)" | 38.0 | 0.03 mass% acrylic acid/vinylpyrrolidone copolymer | 7.0 | 7.2 |
| Comparative Example 13 | "ACUVUE OASYS (registered trademark)" | 38.0 | 0.03 mass% acrylic acid/2-hydroxyethyl methacrylate/N,N-dimethylacrylamide copolymer | 7.0 | 7.1 |
| Comparative Example 14 | "ACUVUE OASYS (registered trademark)" | 38.0 | 0.03 mass% acrylic acid/2-hydroxyethyl methacrylate/N,N-dimethylacrylamide copolymer | 7.0 | 7.1 |

[Table 4-3]

| | Substrate | Water content of substrate (mass%) | Hydrophilic polymer and concentration of hydrophilic polymer solution | pH Before sterilization | pH After sterilization |
|---|---|---|---|---|---|
| Comparative Example 15 | "ACUVUE OASYS (registered trademark)" | 38.0 | 0.03 mass% polyvinylpyrrolidone | 7.0 | 7.2 |

(continued)

| | Substrate | Water content of substrate (mass%) | Hydrophilic polymer and concentration of hydrophilic polymer solution | pH Before sterilization | pH After sterilization |
|---|---|---|---|---|---|
| Comparative Example 16 | "ACUVUE OASYS (registered trademark)" | 38.0 | 0.03 mass% poly (N, N-dimethylacrylamide) | 7.1 | 7.2 |
| Comparative Example 17 | "ACUVUE OASYS (registered trademark)" | 38.0 | No | 7.0 | 7.2 |
| Comparative Example 18 | Production Example 1 | < 1 | No | 7.0 | 7.0 |
| Comparative Example 19 | "1-DAY ACUVUE (registered trademark)" | 58.0 | No | 7. 0 | 7.1 |
| Comparative Example 20 | PF catheter tube | < 1 | No | 7.0 | 7.1 |
| Comparative Example 21 | "MICTRON (registered trademark)" | < 1 | No | 7.0 | 7.1 |

[Table 5-1]

| | Liquid film retention time (sec) | Liquid film retention time 2 hours after scrubbing and washing (sec) | Water content of medical device (mass%) | Results of elemental composition analysis of hydrophilic polymer layer | Film thickness of hydrophilic polymer layer (nm) | Amount of change of water content (percentage point) | Friction coefficient |
|---|---|---|---|---|---|---|---|
| Comparative Example 1 | D (1 sec) | D (1 sec) | 54.0 | No layer observed | 0 | 0 | 0.355 |
| Comparative Example 2 | D (1 sec) | D (1 sec) | 54.0 | No layer observed | 0 | 0 | 0.342 |
| Comparative Example 3 | D (1 sec) | D (1 sec) | 54.1 | No layer observed | 0 | 0. 1 | 0.339 |
| Comparative Example 4 | D (1 sec) | D (1 sec) | 54.1 | No layer observed | 0 | 0. 1 | 0.349 |
| Comparative Example 5 | D (4 sec) | D (1 sec) | 54.1 | No layer observed | 0 | 0. 1 | 0.351 |
| Comparative Example 6 | D (4 sec) | D (1 sec) | 54.0 | No layer observed | 0 | 0 | 0.350 |
| Comparative Example 7 | D (1 sec) | D (1 sec) | 38.0 | No layer observed | 0 | 0 | 0.250 |

[Table 5-2]

| | Liquid film retention time (sec) | Liquid film retention time 2 hours after scrubbing and washing (sec) | Water content of medical device (mass%) | Results of elemental composition analysis of hydrophilic polymer layer | Film thickness of hydrophilic polymer layer (nm) | Amount of change of water content (percentage point) | Friction coefficient |
|---|---|---|---|---|---|---|---|
| Comparative Example 8 | D (1 sec) | D (1 sec) | 38.0 | No layer observed | 0 | 0 | 0.252 |
| Comparative Example 9 | D (1 sec) | D (1 sec) | 38.0 | No layer observed | 0 | 0 | 0.255 |
| Comparative Example 10 | D (1 sec) | D (1 sec) | 38.0 | No layer observed | 0 | 0 | 0.249 |
| Comparative Example 11 | D (1 sec) | D (1 sec) | 38.0 | No layer observed | 0 | 0 | 0.248 |
| Comparative Example 12 | D (1 sec) | D (1 sec) | 38.0 | No layer observed | 0 | 0 | 0.257 |
| Comparative Example 13 | D (1 sec) | D (1 sec) | 38.0 | No layer observed | 0 | 0 | 0.260 |
| Comparative Example 14 | D (1 sec) | D (1 sec) | 38.0 | No layer observed | 0 | 0 | 0.259 |

[Table 5-3]

| | Liquid film retention time (sec) | Liquid film retention time 2 hours after scrubbing and washing (sec) | Water content of medical device (mass%) | Results of elemental composition analysis of hydrophilic polymer layer | Film thickness of hydrophilic polymer layer (nm) | Amount of change of water content (percentage point) | Friction coefficient |
|---|---|---|---|---|---|---|---|
| Comparative Example 15 | D (1 sec) | D (1 sec) | 38.0 | No layer observed | 0 | 0 | 0.263 |
| Comparative Example 16 | D (1 sec) | D (1 sec) | 38.0 | No layer observed | 0 | 0 | 0.266 |
| Comparative Example 17 | D (1 sec) | D (1 sec) | 38.0 | No layer observed | 0 | 0 | 0.300 |
| Comparative Example 18 | E (less than 1 sec) | E (less than 1 sec) | < 1 | No layer observed | 0 | 0 | 0.850 |
| Comparative Example 19 | A (20 sec) | A (20 sec) | 58.0 | No layer observed | 0 | 0 | 0.460 |
| Comparative Example 20 | E (less than 1 sec) | E (less than 1 sec) | < 1 | No layer observed | 0 | 0 | Not measurable because medical device had tubular shape |

(continued)

| | Liquid film retention time (sec) | Liquid film retention time 2 hours after scrubbing and washing (sec) | Water content of medical device (mass%) | Results of elemental composition analysis of hydrophilic polymer layer | Film thickness of hydrophilic polymer layer (nm) | Amount of change of water content (percentage point) | Friction coefficient |
|---|---|---|---|---|---|---|---|
| Comparative Example 21 | E (less than 1 sec) | E (less than 1 sec) | < 1 | No layer observed | 0 | 0 | Not measurable because medical device had sheet shape |

[Table 6-1]

| | Lipid adhesion amount | Tensile elastic modulus of substrate (MPa) | Tensile elastic modulus of medical device (MPa) | Tensile elastic modulus change rate (%) | Size of substrate (mm) | Size of medical device (mm) | Size change rate (% ) |
|---|---|---|---|---|---|---|---|
| Comparative Example 1 | Adhered to 1/5 of total area | 0.61 | Not measured | Not measured | 14.20 | Not measured | Not measured |
| Comparative Example 2 | Adhered to 1/5 of total area | 0.61 | 0.60 | -1.1 | 14.20 | 14.20 | 0 |
| Comparative Example 3 | Adhered to 1/5 of total area | 0.61 | 0.61 | 0.1 | 14.20 | 14.19 | -0.07 |
| Comparative Example 4 | Adhered to 1/5 of total area | 0.61 | 0.61 | 0.3 | 14.20 | 14.17 | -0.20 |
| Comparative Example 5 | Adhered to 1/5 of total area | 0.61 | 0.61 | 0.2 | 14.20 | 14.19 | -0.07 |
| Comparative Example 6 | Adhered to 1/5 of total area | 0.61 | 0.61 | 0.2 | 14.20 | 14.21 | 0.07 |
| Comparative Example 7 | Adhered to 1/5 of total area | 0.70 | 0.73 | 4.3 | 14.20 | 14.21 | 0.07 |

[Table 6-2]

| | Lipid adhesion amount | Tensile elastic modulus of substrate (MPa) | Tensile elastic modulus of medical device (MPa) | Tensile elastic modulus change rate (%) | Size of substrate (mm) | Size of medical device (mm) | Size change rate (% ) |
|---|---|---|---|---|---|---|---|
| Comparative Example 8 | Adhered to 1/5 of total area | 0.71 | 0.70 | -1.3 | 14.20 | 14.20 | 0 |
| Comparative Example 9 | Adhered to 1/5 of total area | 0.71 | 0.70 | -1.4 | 14.20 | 14.19 | -0.07 |
| Comparative Example 10 | Adhered to 1/5 of total area | 0.71 | 0.71 | 0.16 | 14.20 | 14.22 | 0.14 |
| Comparative Example 11 | Adhered to 1/5 of total area | 0.71 | 0.71 | 0.16 | 14.20 | 14.21 | 0.07 |
| Comparative Example 12 | Adhered to 1/5 of total area | 0.71 | 0.70 | -2.4 | 14.20 | 14.20 | 0.04 |
| Comparative Example 13 | Adhered to 1/5 of total area | 0.71 | 0.70 | -1.1 | 14.20 | 14.20 | 0.01 |
| Comparative Example 14 | Adhered to 1/5 of total area | 0.71 | 0.71 | 0.8 | 14.20 | 14.21 | 0.07 |

[Table 6-3]

| | Lipid adhesion amount | Tensile elastic modulus of substrate (MPa) | Tensile elastic modulus of medical device (MPa) | Tensile elastic modulus change rate (%) | Size of substrate (mm) | Size of medical device (mm) | Size change rate (%) |
|---|---|---|---|---|---|---|---|
| Comparative Example 15 | Adhered to 1/5 of total area | 0.71 | 0. 71 | 0.9 | 14.20 | 14.22 | 0.14 |
| Comparative Example 16 | Adhered to 1/5 of total area | 0.71 | 0. 71 | 0.7 | 14.20 | 14.23 | 0.21 |
| Comparative Example 17 | Adhered to 1/5 of total area | 0.71 | 0. 71 | 0 | 14.20 | 14.20 | 0 |
| Comparative Example 18 | Adhered to whole surface | 0.53 | 0.53 | 0 | 14.20 | 14.20 | 0 |
| Comparative Example 19 | No adhesion | 0.30 | 0.30 | 0 | 14.20 | 14.20 | 0 |

(continued)

| | Lipid adhesion amount | Tensile elastic modulus of substrate (MPa) | Tensile elastic modulus of medical device (MPa) | Tensile elastic modulus change rate (%) | Size of substrate (mm) | Size of medical device (mm) | Size change rate (%) |
|---|---|---|---|---|---|---|---|
| Comparative Example 20 | Adhered to whole surface | Not measurable because medical device had tubular shape | Not measurable because medical device had tubular shape | 0 | Not measurable because medical device had tubular shape | Not measurable because medical device had tubular shape | Not measurable because medical device had tubular shape |
| Comparative Example 21 | Adhered to whole surface | 1.10 | 1. 10 | 0 | 15.00 | 15.00 | 0 |

## Claims

1. A method for manufacturing a medical device, the method comprising a step of heating an aqueous packaging solution,

   wherein the heating step is performed in a state in which the aqueous packaging solution contains at least one type of hydrophilic polymer, and a substrate of the medical device is at least partially in contact with the aqueous packaging solution, and
   the method satisfies all of requirements (a) to (c) shown below:

   (a) the hydrophilic polymer is a (co)polymer of a (meth)acrylamide having two substituents, each having 2 or more carbon atoms, on a nitrogen atom;
   (b) the aqueous packaging solution has a mass% concentration of the hydrophilic polymer in a range of 0.0001 to 30 mass%; and
   (c) the aqueous packaging solution has a pH in a range of 6.1 to 8.0 after the heating step.

2. The method according to claim 1, wherein the hydrophilic polymer is a copolymer of the (meth)acrylamide having two substituents, each having 2 or more carbon atoms, on a nitrogen atom with (meth)acrylic acid.

3. The method according to claim 2, wherein the copolymer of the (meth)acrylamide having two substituents, each having 2 or more carbon atoms, on a nitrogen atom with (meth)acrylic acid has a copolymerization ratio of [mass of a (meth)acrylic acid monomer]/[mass of a (meth)acrylamide monomer having two substituents each having 2 or more carbon atoms on a nitrogen atom] = 1/99 to 99/1.

4. The method according to any one of claims 1 to 3, wherein the heating is performed by autoclave sterilization.

5. The method according to any one of claims 1 to 4, wherein the substrate contains at least one type of material selected from the group consisting of a hydrogel, a silicone hydrogel, a low water content soft material, and a low water content hard material.

6. The method according to claim 5, wherein the hydrogel is selected from the group consisting of tefilcon, tetrafilcon, helfilcon, mafilcon, polymacon, hioxifilcon, alfafilcon, omafilcon, nelfilcon, nesofilcon, hilafilcon, acofilcon, deltafilcon, etafilcon, focofilcon, ocufilcon, phemfilcon, methafilcon, and vilfilcon, wherein the aforementioned designations of the members of the group are United States Adopted Names.

7. The method according to claim 5, wherein the silicone hydrogel is selected from the group consisting of lotrafilcon, galyfilcon, narafilcon, senofilcon, comfilcon, enfilcon, balafilcon, efrofilcon, fanfilcon, somofilcon, samfilcon, olifilcon, asmofilcon, formofilcon, stenfilcon, abafilcon, mangofilcon, riofilcon, sifilcon, larafilcon, and delefilcon, wherein the

aforementioned designations of the members of the group are United States Adopted Names.

**8.** The method according to claim 5, wherein the low water content soft material is a material containing a silicon atom.

**9.** The method according to claim 5, wherein the low water content hard material is a material containing a silicon atom.

**10.** The method according to claim 5, wherein the low water content hard material is polymethyl methacrylate.

**11.** The method according to claim 5, 9, or 10, wherein the low water content hard material is a material selected from the group consisting of neofocon, pasifocon, telefocon, silafocon, paflufocon, petrafocon, and fluorofocon, wherein the aforementioned designations of the members of the group are United States Adopted Names.

**12.** The method according to any one of claims 1 to 11, wherein the medical device is an ophthalmic lens, a skin covering material, a wound covering material, a skin protective material, a drug carrier for skin, an infusion tube, a gas transport tube, a drainage tube, a blood circuit, a covering tube, a catheter, a stent, a sheath biosensor chip, a heart-lung machine, or an endoscope covering material.

**13.** The method according to claim 12, wherein the ophthalmic lens is a contact lens.

**Patentansprüche**

**1.** Verfahren zur Herstellung einer medizinischen Vorrichtung, wobei das Verfahren einen Schritt des Erhitzens einer wässrigen Verpackungslösung umfasst,

wobei der Erhitzungsschritt in einem Zustand durchgeführt wird, in dem die wässrige Verpackungslösung mindestens eine Art von hydrophilem Polymer enthält und ein Substrat der medizinischen Vorrichtung mindestens teilweise in Kontakt mit der wässrigen Verpackungslösung ist, und
das Verfahren alle nachstehend gezeigten Anforderungen (a) bis (c) erfüllt:

(a) das hydrophile Polymer ist ein (Co)polymer eines (Meth)acrylamids mit zwei Substituenten, die jeweils 2 oder mehr Kohlenstoffatome aufweisen, an einem Stickstoffatom;
(b) die wässrige Verpackungslösung weist eine Massen-%-Konzentration des hydrophilen Polymers in einem Bereich von 0,0001 bis 30 Massen-% auf; und
(c) die wässrige Verpackungslösung weist nach dem Erhitzungsschritt einen pH-Wert in einem Bereich von 6,1 bis 8,0 auf.

**2.** Verfahren gemäß Anspruch 1, wobei das hydrophile Polymer ein Copolymer des (Meth)acrylamids mit zwei Substituenten, die jeweils 2 oder mehr Kohlenstoffatome aufweisen, an einem Stickstoffatom mit (Meth)acrylsäure ist.

**3.** Verfahren gemäß Anspruch 2, wobei das Copolymer des (Meth)acrylamids mit zwei Substituenten, die jeweils 2 oder mehr Kohlenstoffatome aufweisen, an einem Stickstoffatom mit (Meth)acrylsäure ein Copolymerisationsverhältnis von [Masse eines (Meth)acrylsäuremonomers]/[Masse eines (Meth)acrylamidmonomers mit zwei Substituenten, die jeweils 2 oder mehr Kohlenstoffatome aufweisen, an einem Stickstoffatom] = 1/99 bis 99/1 aufweist.

**4.** Verfahren gemäß einem der Ansprüche 1 bis 3, wobei das Erhitzen durch Autoklaven-Sterilisation durchgeführt wird.

**5.** Verfahren gemäß einem der Ansprüche 1 bis 4, wobei das Substrat mindestens eine Art von Material enthält, ausgewählt aus der Gruppe, bestehend aus einem Hydrogel, einem Silikonhydrogel, einem weichen Material mit niedrigem Wassergehalt und einem harten Material mit niedrigem Wassergehalt.

**6.** Verfahren gemäß Anspruch 5, wobei das Hydrogel ausgewählt ist aus der Gruppe, bestehend aus Tefilcon, Tetrafilcon, Helfilcon, Mafilcon, Polymacon, Hioxifilcon, Alfafilcon, Omafilcon, Nelfilcon, Nesofilcon, Hilafilcon, Acofilcon, Deltafilcon, Etafilcon, Focofilcon, Ocufilcon, Phemfilcon, Methafilcon und Vilfilcon, wobei die vorgenannten Bezeichnungen der Mitglieder der Gruppe "United States Adopted Names" sind.

**7.** Verfahren gemäß Anspruch 5, wobei das Silikonhydrogel ausgewählt ist aus der Gruppe, bestehend aus Lotrafilcon,

Galyfilcon, Narafilcon, Senofilcon, Comfilcon, Enfilcon, Balafilcon, Efrofilcon, Fanfilcon, Somofilcon, Samfilcon, Olifilcon, Asmofilcon, Formofilcon, Stenfilcon, Abafilcon, Mangofilcon, Riofilcon, Sifilcon, Larafilcon und Delefilcon, wobei die vorgenannten Bezeichnungen der Mitglieder der Gruppe "United States Adopted Names" sind.

**8.** Verfahren gemäß Anspruch 5, wobei das weiche Material mit niedrigem Wassergehalt ein Material ist, das ein Siliziumatom enthält.

**9.** Verfahren gemäß Anspruch 5, wobei das harte Material mit niedrigem Wassergehalt ein Material ist, das ein Siliziumatom enthält.

**10.** Verfahren gemäß Anspruch 5, wobei das harte Material mit niedrigem Wassergehalt Polymethylmethacrylat ist.

**11.** Verfahren gemäß Anspruch 5, 9 oder 10, wobei das harte Material mit niedrigem Wassergehalt ein Material ist, ausgewählt aus der Gruppe, bestehend aus Neofocon, Pasifocon, Telefocon, Silafocon, Paflufocon, Petrafocon und Fluorofocon, wobei die vorgenannten Bezeichnungen der Mitglieder der Gruppe "United States Adopted Names" sind.

**12.** Verfahren gemäß einem der Ansprüche 1 bis 11, wobei die medizinische Vorrichtung eine ophthalmische Linse, ein Hautabdeckmaterial, ein Wundabdeckmaterial, ein Hautschutzmaterial, ein Arzneimittelträger für die Haut, ein Infusionsschlauch, ein Gastransportschlauch, ein Drainageschlauch, ein Blutkreislauf, ein Abdeckschlauch, ein Katheter, ein Stent, ein Hüllenbiosensorchip, eine Herz-Lungen-Maschine oder ein Endoskop-Abdeckmaterial ist.

**13.** Verfahren gemäß Anspruch 12, wobei die ophthalmische Linse eine Kontaktlinse ist.

## Revendications

**1.** Procédé de fabrication d’un dispositif médical, le procédé comprenant une étape de chauffage d’une solution d’emballage aqueuse,

où l’étape de chauffage est effectuée dans un état dans lequel la solution d’emballage aqueuse contient au moins un type de polymère hydrophile et un substrat du dispositif médical est au moins partiellement en contact avec la solution d’emballage aqueuse, et
le procédé satisfait à toutes les exigences (a) à (c) présentées ci-dessous :

(a) le polymère hydrophile est un (co)polymère d’un (méth)acrylamide ayant deux substituants ayant chacun 2 atomes de carbone ou plus sur un atome d’azote ;
(b) la solution d’emballage aqueuse a une concentration en % en masse du polymère hydrophile dans une plage de 0,0001 à 30 % en masse ; et
(c) la solution d’emballage aqueuse a un pH dans une plage de 6,1 à 8,0 après l’étape de chauffage.

**2.** Procédé selon la revendication 1, où le polymère hydrophile est un copolymère du (méth)acrylamide ayant deux substituants ayant chacun 2 atomes de carbone ou plus sur un atome d’azote avec de l’acide (méth)acrylique.

**3.** Procédé selon la revendication 2, où le copolymère du (méth)acrylamide ayant deux substituants ayant chacun 2 atomes de carbone ou plus sur un atome d’azote avec de l’acide (méth)acrylique a un rapport de copolymérisation de [masse d’un monomère d’acide (méth)acrylique]/[masse d’un monomère de (méth)acrylamide ayant deux substituants ayant chacun 2 atomes de carbone ou plus sur un atome d’azote] = 1/99 à 99/1.

**4.** Procédé selon l’une quelconque des revendications 1 à 3, où le chauffage est effectué par stérilisation en autoclave.

**5.** Procédé selon l’une quelconque des revendications 1 à 4, où le substrat contient au moins un type de matériau choisi dans le groupe consistant en un hydrogel, un hydrogel de silicone, un matériau mou à faible teneur en eau et un matériau dur à faible teneur en eau.

**6.** Procédé selon la revendication 5, où l’hydrogel est choisi dans le groupe consistant en le tefilcon, le tétrafilcon, l’helfilcon, le mafilcon, le polymacon, l’hioxifilcon, l’alfafilcon, l’omafilcon, le nelfilcon, le nesofilcon, l’hilafilcon, l’acofilcon, le deltafilcon, l’etafilcon, le focofilcon, l’ocufilcon, le phemfilcon, le méthafilcon et le vilfilcon, où les

désignations ci-dessus des membres du groupe sont des « United States Adopted Names ».

7. Procédé selon la revendication 5, où l'hydrogel de silicone est choisi dans le groupe consistant en le lotrafilcon, le galyfilcon, le narafilcon, le senofilcon, le comfilcon, l'enfilcon, le balafilcon, l'efrofilcon, le fanfilcon, le somofilcon, le samfilcon, l'olifilcon, l'asmofilcon, le formofilcon, le stenfilcon, l'abafilcon, le mangofilcon, le riofilcon, le sifilcon, le larafilcon et le delefilcon, où les désignations ci-dessus des membres du groupe sont des « United States Adopted Names ».

8. Procédé selon la revendication 5, où le matériau mou à faible teneur en eau est un matériau contenant un atome de silicium.

9. Procédé selon la revendication 5, où le matériau dur à faible teneur en eau est un matériau contenant un atome de silicium.

10. Procédé selon la revendication 5, où le matériau dur à faible teneur en eau est du polyméthacrylate de méthyle.

11. Procédé selon la revendication 5, 9 ou 10, où le matériau dur à faible teneur en eau est un matériau choisi dans le groupe consistant en le néofocon, le pasifocon, le telefocon, le silafocon, le paflufocon, le pétrafocon et le fluorofocon, où les désignations ci-dessus des membres du groupe sont des « United States Adopted Names ».

12. Procédé selon l'une quelconque des revendications 1 à 11, où le dispositif médical est une lentille ophtalmique, un matériau de recouvrement de la peau, un matériau de recouvrement de plaie, un matériau de protection de la peau, un support de médicament pour la peau, un tube de perfusion, un tube de transport de gaz, un tube de drainage, un circuit sanguin, un tube de recouvrement, un cathéter, un stent, une puce de biocapteur de gaine, une machine cœur-poumon ou un matériau de recouvrement d'endoscope.

13. Procédé selon la revendication 12, où la lentille ophtalmique est une lentille de contact.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2017146102 A
- JP 2011512546 A
- JP 2003535626 A
- JP 5154231 B
- WO 2017169873 A1
- WO 2013024799 A
- JP 2010088858 A
- WO 2013024800 A
- JP 2014533381 A